# EUROPEAN PATENT SPECIFICATION

(11) **EP 2 618 826 B1**
(45) Date of publication and mention of the grant of the patent: **13.04.2016**
(21) Application number: 11827047.9
(22) Date of filing: 20.09.2011
(51) Int. Cl.: A61K 31/445, A61P 25/28

(54) **PHENYLPIPERIDINE COMPOUNDS FOR THE TREATMENT OF DEMENTIA**
PHENYLPIPERIDINVERBINDUNGEN ZUR BEHANDLUNG VON DEMENZ
COMPOSÉS DE PHÉNYLPIPÉRIDINE POUR LE TRAITEMENT DE LA DEMENCE

(30) Priority: 20.09.2010 US 384403 P
(43) Date of publication of application: 31.07.2013
(73) Proprietor: A.Carlsson Research AB, 413 19 Göteborg (SE)
(72) Inventor: CARLSSON, Lizzie Maria, S-413 23 Göteborg (SE); KLOBERG, Angélica, S-423 50 Torslanda (SE); BURSTEIN, Ethan, S., San Diego, CA 92130 (US); CARLSSON, Per Arvid Emil, S-413 19 Göteborg (SE)
(74) Representative: Awapatent AB
(86) International application number: PCT/SE2011/000163
(87) International publication number: WO 2012/039660

(56) References cited:
- EP-B1- 0 641 320
- WO-A1-00/03714
- WO-A1-01/46145
- WO-A1-99/03470
- WO-A2-01/81343
- WO-A2-02/13807
- WO-A2-02/36123
- WO-A2-2008/155357
- US-A- 5 462 947
- US-A1- 2003 130 313

## Description

### TECHNICAL FIELD

The present invention relates to 3-[3-(methylsulfonyl)phenyl]-1-propylpiperidine or a pharmaceutically acceptable salt thereof for use in the treatment and/or prevention of dementia selected from the group of disorders consisting of Alzheimer's disease, vascular dementia, frontotemporal dementia, semantic dementia and dementia with Lewy bodies.

### BACKGROUND OF THE INVENTION

During the last three decades considerable knowledge about the pharmacology of phenylpiperidines has accumulated. In the 1980s [3-(3-hydroxyphenyl)-N-n-propyl]piperidine (= 3PPP) was prepared and described as a dopamine D2/D3-receptor agonist. The R-enantiomer was found to be a full agonist and the S-enantiomer a partial agonist on these receptors. The latter was brought to testing in schizophrenia as well as Parkinson patients, and its mixed agonist-antagonist properties on dopamine receptors could be clinically verified. Subsequently molecules with electron-withdrawing substituents replacing the hydroxyl group were prepared, and this led to lower affinities and intrinsic activities on the D2/D3 receptors.

Further work in this area focused on the S-enantiomers, and especially the methylsulfonyl derivative (= OSU6162 or PNU-96391). In spite of a very low intrinsic activity, which could be detected *in vitro* but hardly *in vivo*, this compound has retained a pharmacological profile reminiscent of a partial agonist. Thus it is able to act as an antagonist and thus inhibit behavior under conditions of high psychomotor activity, e g in rats exposed to a novel, highly stimulating environment, whereas it moderately but significantly stimulates the behavior of animals which have been given time to become habituated to a less stimulating environment. The mechanisms underlying this dual action are not yet fully clarified. The inhibitory component could well be due to antagonism on postsynaptic dopamine receptors. The mechanism underlying the stimulatory component is less clear, especially in view of the fact that this effect is not shared by established partial dopamine receptor agonists such as (-)-3PPP and aripiprazole.

Preferential autoreceptor antagonism might be involved but also speculations about putative allosteric sites on dopamine receptors or" functional selectivity" have been advanced in this context. In any event the available *in vitro* and *in vivo* binding data support some kind of dopaminergic mechanism, and these novel compounds have thus been described as "dopamine receptor stabilizers", which might be useful in the treatment of conditions characterized by instability of dopaminergic tone. Promising early clinical observations in patients with Parkinson's disease, Huntington's disease and schizophrenia tend to support this notion.

In *in vitro* binding studies (-)-OSU6162 has a relatively low affinity for dopamine D2/D3 receptors (Kᵢ = 447 nM) but an even lower affinity for the large number of other receptors studied so far in this context. Under *in vivo* conditions even relatively low doses of (-)-OSU6162 have the capacity to bind to dopamine receptors, as shown by the displacement of D2-selective ligands (e.g. raclopride) in the striatum. As a dopaminergic ligand it acts as an antagonist although a low intrinsic activity can be demonstrated under in-vitro conditions. Thus its mode of action must be assumed to differ from that of partial dopamine receptor agonists, in spite of their similarity in many respects with regard to behavioral profiles.

So far not much work has been done on the R-enantiomer of OSU6162 or related compounds. For example, in the first patent describing these compounds, R-(+)-OSU6162, although included among the compounds claimed in the patent, had not yet been prepared. One year later, in 1995, Sonesson in his doctoral thesis (Sonesson C (1995) Arylpiperidine and arylpyrrolidine derivatives with potential antipsychotic efficacy. Thesis, ISBN 91-554-3453-3) described the preparation of this compound and presented some data on its pharmacological properties. Together with two congeners, likewise R-enantiomers, it was stated not to induce any change in behavioral activity. The only pharmacological effect of R-(+)-OSU6162 reported in Sonesson's thesis was a weak increase in the synthesis of dopamine in the striatum of the brain. However, the present work will quite contradictory to earlier findings present evidence that:
1) R-(+)-OSU6162 shows strong activity in several different models of animal behavior.
2) Both enantiomers of OSU6162 can influence animal behavior, not only via a dopaminergic, but also via a strong serotonergic component.
3) The two enantiomers have clearly different behavioral profiles showing up in both rats and mice.
4) The behavioral effects of both enantiomers of OSU6162 can be reconciled with their in vitro functional selectivity profiles.

These discoveries have important implications for the potential clinical utility of both compounds, as well as for several of their congeners.
WO99/03470 discloses specific substituted 3-phenylpiperidines and 3-phenylpyrrolidine analogs useful in treatment of cognitive disorders.
WO01/46145 discloses a 4-(3-methylsulfonyl)phenyl substituted propylpiperidine compound for treatment of disorders in the central nervous system.

### SUMMARY OF THE INVENTION

The present invention relates to 3-[3-(methylsulfonyl)phenyl]-1-propylpiperidine or a pharmaceutically acceptable salt thereof for use in the treatment and/or prevention of dementia selected from the group of disorders consisting of Alzheimer's disease, vascular dementia, frontotemporal dementia, semantic dementia and dementia with Lewy bodies, wherein 3-[3-(methylsulfonyl)phenyl]-1-propylpiperidine or the pharmaceutically acceptable salt thereof acts as a partial agonist on the 5-HT receptor.
The at least one 5-hydroxytryptamine receptor (5-HT receptor) may be of the group consisting of 5HT₁, 5HT₂, 5HT₃, 5HT₄, 5HT₅, 5HT₆, and 5HT₇ receptors, such as for example one or more of the 5HT_{1A}, 5HT_{1B}, 5HT_{1D}, 5HT_{1E}, 5HT_{1F}, 5HT_{2A}, 5HT_{2B}, 5HT_{2C}, 5HT₃, 5HT₄, 5HT_{5A}, 5HT₆, and 5HT₇ receptors. In one embodiment the at least one 5-hydroxytryptamine receptor (5-HT receptor) may be a 5HT₂ receptor, such as for example one or more of the group consisting of the receptors 5HT_{2A}, 5HT_{2B}, and 5HT_{2C}, and especially the receptors 5HT_{2A} and/or 5HT_{2B}.

The monoaminergic neurotransmitters receptor may be one or more 5-hydroxytryptamine receptor (5-HT receptor).

The one or more of the monoaminergic neurotransmitter receptors may comprise a dopamine receptor (DA receptor). Such dopamine receptor may be of the group consisting of D₁, D₂, D₃, D₄, and D₅ receptors. In one embodiment the dopamine receptor may be a D₂ receptor.

The one or more diseases associated with a need for modulation of one or more monoaminergic neurotransmitter receptors is selected from the group consisting of depression, dementia, cognitive dysfunctions, aggressive behavior, impulsive behavior obsessive-compulsive disorder (OCD) and anxiety disorders.

The disease depression may be selected from the group of disorders consisting of recurrent depressive disorders, clinical depression, major depression, unipolar depression, and unipolar disorders.

The disease dementia may be selected from the group of disorders consisting of Alzheimer's disease, vascular dementia, frontotemporal dementia, semantic dementia and dementia with Lewy bodies.

The diseases cognitive dysfunctions may be selected from the group of disorders consisting of Alzheimer's disease, Parkinson's disease and chronic alcoholism, heavy metal poisoning, menopause, fibromyalgia, mood disorders, Attention-deficit Disorders (ADD, ADHD) and sleep disorders.

The disease impulsive behavior may be selected from the group of disorders consisting of trichotillomania, intermittent explosive disorder, pathological gambling, kleptomaniahttp://www.forensicpsychiatry.ca/forensic/Criminology/impulse/gambling.htm and pyromania.

The anxiety disorders may be selected from the group of disorders consisting of panic disorder, agoraphobia, social phobia, phobias, general anxiety disorder, posttraumatic stress disorder, and premenstrual tension.

At least one disease associated with a need for modulation of at least one dopamine receptor may be selected from the group consisting of neurological and psychiatric disorders characterized by a dysfunction of the dopamine system.

Such neurological and psychiatric disorders may be selected from the group of disorders consisting of Parkinson's disease in early stages, restless legs, akathisia, dystonias, mental fatigue associated with high age, stroke, postencephalitic or posttraumatic conditions, attention-deficit disorders (ADHD and ADD), autism spectrum disorders, lapses of consciousness including narcolepsy, petit mal epilepsy and syncope, sleeping disorders including hypersomnia, sleep apnea, and attacks of sleep induced by dopamine receptor agonists, dopamine hypofunction induced by antipsychotic drugs, Tourette's syndrome, and chronic fatigue syndrome (CFS).

### BRIEF DESCRIPTION OF THE DRAWINGS

Figure 1. Effects of (-)-OSU6162 and (+)-OSU6162 on motor activity in reserpine-pretreated mice.
Figure 2. Effects of different monoamine receptor antagonists on the locomotor stimulation induced by (-)-OSU6162 or (+)-OSU6162 in reserpine-pretreated mice.
Figure 3. Effects of various doses of M100907 on the locomotor stimulation induced by (-)-OSU6162 or (+)-OSU6162 in reserpine-pretreated mice.
Figure 4. Locomotor-inducing effects of DOI in reserpine-pretreated mice.
Figure 5. Head twitch-inducing effects of DOI, (-)-OSU6162 and (+)-OSU6162 in mice.
Figure 6. Antagonizing effects of (-)-OSU6162 and (+)-OSU6162 on DOI-induced head twitch response in mice.
Figure 7. Effects of (-)-OSU6162 and (+)-OSU6162 on motor activity in mice.
Figure 8. Effects of (-)-OSU6162 or (+)-OSU6162 on motor activity in active rats.
Figure 9. Effects of (-)-OSU6162 and (+)-OSU6162 on locomotion in habituated rats.
Figure 10. Effect of haloperidol on the locomotor stimulation induced by a) (-)-OSU6162 and b) (+)-OSU6162 in habituated rats.
Figure 11. Effect of M100907 on the locomotor stimulation induced by a) (-)-OSU6162 and b) (+)-OSU6162 in habituated rats.
Figure 12. 5-HT2A R-SAT^{™} functional assays.
Figure 13. 5-HT2A phosphatidyl inositol (PI) hydrolysis assays.
Figure 14. 5-HT2A Bioluminescence Resonance Energy Transfer (BRET2) assays.
Figure 15. D2 R-SAT^{™} functional assays.
Figure 16. D2 GTPγS binding assays.
Figure 17. D2 Bioluminescence Resonance Energy Transfer (BRET2) assays.
Figure 18. Competitive functional antagonism of dopamine by (-)-OSU6162.

### DETAILED DESCRIPTION OF THE INVENTION

Before the present invention is described, it is to be understood that this invention is not limited to the particular embodiments described, as such formulations may, of course, vary. It is also to be understood that the terminology used herein is for the purpose of describing particular embodiments only, and is not intended to limit the scope of the present invention which will be limited only by the appended claims. It must be noted that as used herein and in the appended claims, the singular forms "a," "an," and "the" include plural referents unless the context clearly dictates otherwise, and includes reference to equivalent steps and methods known to those skilled in the art.

Although any methods and materials similar or equivalent to those described herein can be used in the practice or testing of the present invention, the preferred methods and materials are now described. All publications mentioned herein are incorporated herein by reference to disclose and describe the specific methods and/or materials in connection with which the publications are cited. Unless defined otherwise, all technical and scientific terms used herein have the same meaning as commonly understood by one of ordinary skill in the art to which this invention belongs. A few exceptions, as listed below, have been further defined within the scope of the present invention.

As used herein the terms "agonist" or "receptor agonist" are intended to mean a chemical that binds to a receptor of a cell and triggers a response by that cell. Agonists often mimic the action of a naturally occurring substance. Full agonists bind (have affinity for) and activate a receptor, displaying full efficacy at that receptor, while partial agonists also bind and activate a given receptor, but have only partial efficacy at the receptor relative to a full agonist. Partial agonists can act as a competitive antagonist in the presence of a full agonist, as it competes with the full agonist for receptor occupancy, thereby producing a net decrease in the receptor activation as compared to that observed with the full agonist alone. An endogenous agonist for a particular receptor is a compound naturally produced by the host that binds to and activates that receptor.

As used herein the terms "antagonist" or "receptor antagonist" are intended to mean a receptor ligand or drug that does not provoke a biological response itself upon binding to a receptor, but blocks or dampens agonist-mediated responses. The antagonists have affinity but no efficacy for their cognate receptors, and binding will disrupt the interaction and inhibit the function of an agonist or inverse agonist at receptors. Antagonists mediate their effects by binding to the active site or to allosteric sites on receptors, or they may interact at unique binding sites not normally involved in the biological regulation of the receptor's activity. Antagonist activity may be reversible or irreversible depending on the longevity of the antagonist-receptor complex, which, in turn, depends on the nature of antagonist receptor binding. Thus, drug antagonists achieve their potency by competing with endogenous ligands or substrates at structurally-defined binding sites on receptors. Antagonists display no efficacy to activate the receptors they bind. Once bound, however, antagonists inhibit the function of agonists, inverse agonists, and partial agonists.

As used herein the terms "monoamines" or "monoamine neurotransmitters" refer to neurotransmitters and neuromodulators that contain one amino group that is connected to an aromatic ring by a two-carbon chain (-CH₂-CH₂-). Examples of monoamines are catecholamines: eg. epinephrine (adrenaline), norepinephrine (noradrenaline) and dopamine; tryptamines: eg. serotonin and melatonin; and trace amines.

As used herein the term "dopamine" (sometimes abbreviated DA) refers to a catecholamine neurotransmitter present in a wide variety of animals, including both vertebrates and invertebrates. In the brain, this substituted phenethylamine functions as a neurotransmitter, activating the five known types of dopamine receptors: D₁, D₂, D₃, D₄, and D₅, and their variants. Furthermore, the term "dopaminergic" means related to the neurotransmitter dopamine.

As used herein the term "dopamine stabilizer" is intended to mean a substance that normalizes dopaminergic transmission in case of either excessive or deficient signaling. Such drugs may be useful but limited to treating conditions involving both increased and decreased dopaminergic tone.

As used herein the term "dopamine transporter" or "DAT", refers to a membrane-spanning protein that pumps the neurotransmitter dopamine out of the synapse back into cytosol. DAT is a symporter that moves dopamine across the cell membrane by coupling the movement to the energetically-favorable movement of sodium ions moving from high to low concentration into the cell. DAT function requires the sequential binding and co-transport of two Na⁺ ions and one Cl⁻ ion with the dopamine substrate.

As used herein the terms "serotonin" or "5-hydroxytryptamine (5-HT)" refer to a monoamine neurotransmitter. Biochemically derived from tryptophan, serotonin is primarily found in the gastrointestinal (GI) tract, platelets, and in the central nervous system (CNS) of animals including humans. Furthermore, the term "serotonergic" means related to the neurotransmitter serotonin.

When used herein the terms "Selective serotonin re-uptake inhibitors" or "serotonin-specific reuptake inhibitor (SSRIs)" refer to a class of compounds typically used as antidepressants in the treatment of depression, anxiety disorders, and some personality disorders. SSRIs are believed to increase the extracellular level of the neurotransmitter serotonin by inhibiting its reuptake into the presynaptic cell, increasing the level of serotonin in the synaptic cleft available to bind to the postsynaptic receptor. They have varying degrees of selectivity for the other monoamine transporters, with pure SSRIs having only weak affinity for the noradrenaline and dopamine transporter.

As used herein the terms "serotonin transporter" or "SERT", refer to a monoamine transporter protein. It is an integral membrane protein that transports the neurotransmitter serotonin from synaptic spaces into presynaptic neurons. This transport of serotonin by the SERT protein terminates the action of serotonin and recycles it in a sodium-dependent manner.

As used herein the terms "norepinephrine transporter" or "NET" refer to a monoamine transporter that transports the neurotransmitters norepinephrine (noradrenaline) and dopamine from the synapse back to cytosol,

The term "adrenergic receptors" when used herein, refers to a class of metabotropic G protein-coupled receptors that are targets of the catecholamines, especially noradrenaline (norepinephrine) and adrenaline (epinephrine). Included are the noradrenaline and the adrenaline receptors.

As used herein the term "orthosteric site" is intended to mean the region of the receptor to which the endogenous agonist binds.

As used herein the term "allosteric site" is intended to mean a site on a receptor (or a multi-subunit enzyme) that is not the endogenous agonist binding site.

As used herein "intrinsic activity" or "efficacy" refer to the relative ability of a drug-receptor complex to produce a maximum functional response. High efficacy agonists can produce the maximal response of the receptor system while occupying a relatively low proportion of the receptors in that system. Agonists of lower efficacy are not as efficient at producing a response from the drug-bound receptor, by stabilizing the active form of the drug-bound receptor. Therefore, they may not be able to produce the same maximal response, even when they occupy the entire receptor population, as the efficiency of transformation of the inactive form of the drug-receptor complex to the active drug-receptor complex may not be high enough to evoke a maximal response.

As used herein the term "half maximal effective concentration" or "EC₅₀" refers to the concentration of a drug, antibody or toxicant which induces a response halfway between the baseline and maximum after some specified exposure time. It is commonly used as a measure of drug's potency. The EC₅₀ of a graded dose response curve therefore represents the concentration of a compound where 50% of its maximal effect is observed.

The following drugs have in the context of this application the following effects:
Haloperidol is a selective DA D2 receptor antagonist;
M100907 is a selective 5-HT2A receptor antagonist;
Raclopride is a selective DA D2 receptor antagonist;
Reserpine mediates depletion of monoamine neurotransmitters from peripheral and central nervous monoaminergic nerve cells. It acts by blocking the vesicular monoamine transporter VMAT which normally transports free norepinephrine, serotonin, and dopamine from the cytoplasm of the presynaptic nerve terminal into storage vesicles for subsequent release into the synaptic cleft;
SCH23390 is a selective DA D1 receptor antagonist and has either minimal or negligible effects on the D₂ receptor;
SCH39166 is a selective DA D1 receptor antagonist;
DOI (dimetoxiamfetamin) is a 5-HT_{2A}, 5-HT_{2B}, and 5-HT_{2C} receptor agonist. Its psychedelic effects are mediated by its agonistic properties at the 5-HT_{2A} receptor;
NDMC (N-desmethylclozapine) acts as a weak partial agonist at the D₂/D₃ receptors;
Aripiprazole acts as a D₂ partial agonist. Aripiprazole is also a partial agonist at the 5-HT1A receptor, and like the other atypical antipsychotics displays an antagonist profile at the 5-HT2A receptor. It also antagonizes the 5-HT7 receptor and acts as a partial agonist at the 5-HT2C receptor, both with high affinity;
Bifeprunox combines minimal D₂ receptor agonism with 5-HT receptor agonism; (-)-3-PPP is a D2 partial agonist;
Pramipexol acts as a partial/full agonist at the following receptors:D_{2S} receptor, D_{2L} receptor, D₃ receptor, D₄ receptor. Pramipexole also possesses low/insignificant affinity for the 5-HT_{1A}, 5-HT_{1B}, 5-HT_{1D}, and α₂ adrenergic receptors;
Roxindol, acts as an agonist at the D2, D3, D4 and 5HT1A receptors, but has been reported to act as a 5-HT2A receptor antagonist as well;
Pergolide functions as an agonist at the dopamine D2, D1 and serotonin 5-HT1A, 5-HT1 B, 5-HT2A, 5-HT2B, and 5-HT2C receptors;
Talipexole is a D2 full agonist;
Quinelorane is a D2 full agonist;
Ropinirole acts as a D2, D3, and D4 dopamine receptor agonist with highest affinity for D₃. It is weakly active at the 5-HT2, and α₂ receptors and is said to have virtually no affinity for the 5-HT1, benzodiazepine, GABA, muscarinic, α₁-, and β-adrenoreceptors;
Desipramine inhibits the reuptake of norepinephrine and to a lesser extent serotonin;
Amoxapine is a strong norepinephrine reuptake inhibitor and weak serotonin reuptake inhibitor. It also possesses antiadrenergic, anticholinergic, antidopaminergic, antihistamine, and antiserotonergic actions;
Fluoxetine is an antidepressant of the selective serotonin reuptake inhibitor (SSRI) class; Buproprion, acts as a norepinephrine reuptake inhibitor and nicotinic acetylcholine receptor antagonist;
Indatraline, is a non-selective monoamine transporter inhibitor that has been shown to block the reuptake of dopamine, norepinephrine, and serotonin;
Mazindol is thought to act as a reuptake inhibitor of norepinephrine. In addition, it inhibits dopamine and serotonin reuptake;
Terguride (INN) is a dopamine agonist;
Quinpirole acts as a selective D₂ and D₃ receptor agonist

As used herein the term "drug naïve mice" refers to mice not previously treated with a drug.

As used herein the term "monoamine-depleted mice" is intended to mean mice that have been depleted of monoaminergic stores by injecting reserpine intraperitoneally (i.p.) as pretreatment prior to an activity recording. After such treatment the mice normally exhibit strongly reduced motility and wakefulness.

"Low activity" animals are rats or mice which have been given time to become habituated to a less stimulating environment.

"High activity" animals are rats or mice exposed to a novel, highly stimulating environment The term "subject" includes, but is not limited to, humans, nonhuman primates such as chimpanzees and other apes and monkey species, farm animals such as cattle, sheep, pigs, goats and horses, domestic mammals such as dogs and cats, laboratory animals including rodents such as mice, rats and guinea pigs, and the like. The term does not denote a particular age or sex. Thus, adult and newborn subjects, as well as fetuses, whether male or female, are intended to be covered. In preferred embodiments, the subject is a mammal, including humans and non-human mammals. In the most preferred embodiment, the subject is a human.

As used herein the term "treatment" includes the attempted prevention, remediation, amelioration, and the prevention of relapse of a health problem in a subject, usually following a diagnosis.

The invention will now be described in more detail. However, the described embodiments mentioned below are only given as examples and should not be limiting to the present invention. Other solutions, uses, objectives, and functions within the scope of the invention as claimed in the below described patent claims should be apparent for the person skilled in the art.

More precisely, the present invention refers to 3-[3-(methylsulfonyl)phenyl]-1-propylpiperidine or a pharmaceutically acceptable salt thereof for use in the treatment and/or prevention of dementia selected from the group of disorders consisting of Alzheimer's disease, vascular dementia, frontotemporal dementia, semantic dementia and dementia with Lewy bodies, wherein 3-[3-(methylsulfonyl)phenyl]-1-propylpiperidine or the pharmaceutically acceptable salt thereof acts as a partial agonist on the 5-HT receptor.

Also disclosed herein is the use of a compound selected from the group consisting of:

### compounds of formula I

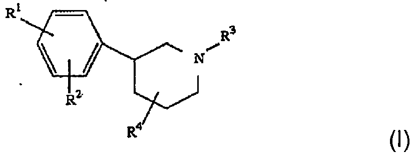

wherein:
R¹ and R² are independently selected from the group consisting of H (provided that not more than one of R¹ and R² is H), CONH₂, OH, CN, CH₂CN, OSO₂CH₃, OSO₂CF₃, SSO₂CF₃, COR, SOₓCH₃ (where x is 0-2), SOₓCF₃, O(CH₂)ₓCF₃, OSO₂N(R)₂, CH=NOR, COCOOR, COCOON(R)₂, C₃₋₈ cycloalkyl, NRSO₂CF₃, phenyl at position 2, 3 or 4, thienyl, furyl, pyrrolyl, oxazolyl, thiazolyl, N-pyrrolinyl, triazolyl, tetrazolyl of pyridinyl;
R³ is hydrogen, CF₃, CH₂CF₃, C₁-C₈ alkyl, C₃-C₈ cycloalkyl, C₄-C₉ cycloalkyl-methyl, C₂-C₈ alkenyl, C₂-C₈ alkynyl, 3,3,3-trifluoropropyl, 4,4,4-trifluorobutyl, or CH₂SCH₃,
R⁴ and R are independently selected from hydrogen, CF₃CH₂CF₃, C₁-C₈ alkyl, C₃-C₈ cycloalkyl, C₄-C₉ cycloalkyl-methyl, C₁-C₈ alkenyl, C₂-C₈ alkynyl, 3,3,3-trifluoropropyl, 4,4,4-trifluorobutyl or -(CH₂)ₘ-R⁵ where m is 1-8;
R⁵ is phenyl, phenyl substituted with CN, CF₃, CH₂CF₃, C₁ -C₈ alkyl, C₃ -C₈ cycloalkyl, C₄-C₉ cycloalkyl-methyl, C₂-C₈ alkenyl or C₂-C₈ alkynyl substituent, 2-thiophenyl, 3-thiophenyl, -NR⁶CONR⁶R⁷ or -CONR⁶R⁷; and
R⁶ and R⁷ are independently H, C₁-C₈ alkyl, C₃-C₈ cycloalkyl, C₄-C₉ cycloalkyl-methyl, C₂-C₈alkenyl or C₂-C₈ alkynyl,
or a suitable pharmaceutically acceptable salt thereof;
for the treatment and/or prevention of one or more diseases associated with a need for modulation of one or more monoaminergic neurotransmitter receptors, characterized in that at least one of the monoaminergic neurotransmitter receptors with a need for modulation is a 5-hydroxytryptamine receptor (5-HT receptor), and in that said compound of formula I acts as a partial agonist on the one or more monoaminergic neurotransmitter receptors.

Also disclosed herein is the use of a compound selected from the group consisting of:

### compounds of formula I

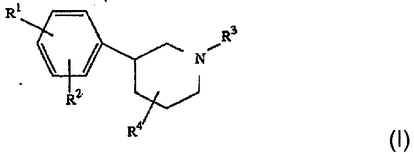

wherein:
R¹ and R² are independently selected from the group consisting of H (provided that not more than one of R¹ and R² is H), CONH₂, OH, CN, CH₂CN, OSO₂CH₃, OSO₂CF₃, SSO₂CF₃, COR, SOₓCH₃ (where x is 0-2), SOₓCF₃, O(CH₂)ₓCF₃, OSO₂N(R)₂, CH=NOR, COCOOR, COCOON(R)₂, C₃₋₈cycloalkyl, NRSO₂CF₃, phenyl at position 2, 3 or 4, thienyl, furyl, pyrrolyl, oxazolyl, thiazolyl, N-pyrrolinyl, triazolyl, tetrazolyl of pyridinyl;
R³ is hydrogen, CF₃, CH₂CF₃, C₁-C₈ alkyl, C₃-C₈ cycloalkyl, C₄-C₉ cycloalkyl-methyl, C₂-C₈ alkenyl, C₂-C₈ alkynyl, 3,3,3-trifluoropropyl, 4,4,4-trifluorobutyl, or CH₂SCH₃,
R⁴ and R are independently selected from hydrogen, CF₃CH₂CF₃, C₁-C₈ alkyl, C₃-C₈ cycloalkyl, C₄-C₉ cycloalkyl-methyl, C₂-C₈ alkenyl, C₂-C₈ alkynyl, 3,3,3-trifluoropropyl, 4,4,4-trifluorobutyl or -(CH₂)ₘ-R⁵ where m is 1-8;
R⁵ is phenyl, phenyl substituted with CN, CF₃, CH₂CF₃, C₁ -C₈ alkyl, C₃ -C₈ cycloalkyl, C₄-C₉ cycloalkyl-methyl, C₂-C₈ alkenyl or C₂-C₈ alkynyl substituent, 2-thiophenyl, 3-thiophenyl, -NR⁶CONR⁶R⁷ or -CONR⁶R⁷; and
R⁶ and R⁷ are independently H, C₁-C₈ alkyl, C₃-C₈ cycloalkyl, C₄-C₉ cycloalkyl-methyl, C₂-C₈ alkenyl or C₂-C₈ alkynyl,
or a suitable pharmaceutically acceptable salt thereof;
for the manufacture of a medicament for the treatment and/or prevention of one or more diseases associated with a need for modulation of one or more monoaminergic neurotransmitter receptors, characterized in that at least one of the monoaminergic neurotransmitter receptors with a need for modulation is a 5-hydroxytryptamine receptor (5-HT receptor), and in that said compound of formula I acts as a partial agonist on the one or more monoaminergic neurotransmitter receptors.

Further disclosed herein is a method of treating and/or preventing at least one disease in a subject, said disease being associated with a need for modulation of one or more monoaminergic neurotransmitter receptors, characterized in that at least one of the monoaminergic neurotransmitter receptors with a need for modulation is a 5-hydroxytryptamine receptor (5-HT receptor), and in that said compound of formula I acts as a partial agonist on the one or more monoaminergic neurotransmitter receptors, said method comprising the administration of a therapeutically effective amount of a compound selected from the group consisting of:

### compounds of formula I

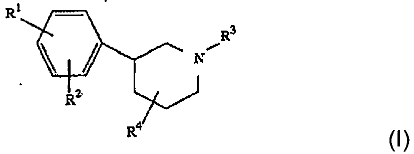

wherein:
R¹ and R² are independently selected from the group consisting of H (provided that not more than one of R¹ and R² is H), CONH₂, OH, CN, CH₂CN, OSO₂CH₃, OSO₂CF₃, SSO₂CF₃, COR, SOₓCH₃ (where x is 0-2), SOₓCF₃, O(CH₂)ₓCF₃, OSO₂N(R)₂, CH=NOR, COCOOR, COCOON(R)₂, C₃₋₈ cycloalkyl, NRSO₂CF₃, phenyl at position 2, 3 or 4, thienyl, furyl, pyrrolyl, oxazolyl, thiazolyl, N-pyrrolinyl, triazolyl, tetrazolyl of pyridinyl;
R³ is hydrogen, CF₃, CH₂CF₃, C₁-C₈ alkyl, C₃-C₈ cycloalkyl, C₄-C₉ cycloalkyl-methyl, C₂-C₈ alkenyl, C₂-C₈ alkynyl, 3,3,3-trifluoropropyl, 4,4,4-trifluorobutyl, or CH₂SCH₃,
R⁴ and R are independently selected from hydrogen, CF₃CH₂CF₃, C₁-C₈ alkyl, C₃-C₈ cycloalkyl, C₄-C₉ cycloalkyl-methyl, C₂-C₈ alkenyl, C₂-C₈ alkynyl, 3,3,3-trifluoropropyl, 4,4,4-trifluorobutyl or -(CH₂)ₘ-R⁵ where m is 1-8;
R⁵ is phenyl, phenyl substituted with CN, CF₃, CH₂CF₃, C₁ -C₈ alkyl, C₃ -C₈ cycloalkyl, C₄-C₉ cycloalkyl-methyl, C₂-C₈ alkenyl or C₂-C₈ alkynyl substituent, 2-thiophenyl, 3-thiophenyl, -NR⁶CONR⁶R⁷ or -CONR⁶R⁷ ; and
R⁶ and R⁷ are independently H, C₁-C₈ alkyl, C₃-C₈ cycloalkyl, C₄-C₉ cycloalkyl-methyl, C₂-C₈ alkenyl or C₁-C₈ alkynyl,
or a suitable pharmaceutically acceptable salt thereof;
to a subject in need thereof.

The US Patent 5,462,947, more specifically in column 7, lines 26-28 and the Examples disclose how the compounds of Formula I can be obtained. It is important to note that the compounds of the present invention also encompass any pharmaceutically acceptable salts of the same compound. A suitable pharmaceutically acceptable salt of a compound of the invention is, for example, an acid-addition salt of a compound of the invention which is sufficiently basic, for example, an acid-addition salt with, for example, an inorganic or organic acid, for example hydrochloric, hydrobromic, nitric, methansulphonic, sulphuric, phosphoric, trifluoroacetic, para-toluene sulphonic, 2-mesitylen sulphonic, citric, acetic, tartaric, fumaric, lactic, succinic, malic, malonic, maleic, 1,2-ethanedisulphonic, adipic, aspartic, benzenesulphonic, benzoic, ethanesulphonic or nicotinic acid. In addition a suitable pharmaceutically acceptable salt of a compound of the invention, is, for example, a base-addition salt of a compound of the invention which is sufficiently acidic, for example, a metal salt, for example, sodium, potassium, calcium, magnesium, zinc or aluminum, an ammonium salt, a salt with an organic base which affords a physiologically acceptable cation, which includes quartenery ammonium hydroxides, for example methylamine, ethylamine, diethylamine, trimethylamine, tert- butylamine, triethylamine, dibenzylamine, N,N-dibenzylethylamine, cyclohexylethylamine, tris-(2-hydroxyethyl)amine, hydroxyethyl diethylamine, (IR, 2S)-2-hydroxyinden-I -amine, morpholine, N-methylpiperidine, N-ethylpiperidine, piperazine, methylpiperazine, adamantylamine, choline hydroxide, tetrabutylammonium hydroxide, tris-(hydroxymethyl)methylamine hydroxide, L-arginine, N-methyl D-glucamine, lysine or arginine.

Certain compounds of the present invention may exist as tautomers or stereoisomers (e.g. racemate, enantiomer, diastereomer or E- or Z-isomer). It is to be understood that the present invention encompasses all such tautomers or stereoisomers. Furthermore, certain compounds of the present invention may exist as solvates or hydrates. It is to be understood that the present invention encompasses all such solvates or hydrates.

Said compounds of Formula I may be used for the treatment and/or prevention of one or more diseases associated with a need for modulation of one or more monoaminergic neurotransmitter receptors, characterized in that at least one of the monoaminergic neurotransmitter receptors with a need for modulation is a 5-hydroxytryptamine receptor (5-HT receptor), and in that said compound of formula I acts as a partial agonist on the one or more monoaminergic neurotransmitter receptors.

As used herein the term "monoaminergic neurotransmitter receptor" refers to a group of G-protein linked receptors expressed on the surface of post-synaptic cells indirectly linked to ion channels, via a second messenger system involving G-proteins and adenylate cyclase. They are also expressed on pre-synaptic cells to provide feedback mechanisms and attenuate excessive neurotransmitter release. Binding of a ligand to its specific neurotransmitter receptor may result in the activation of a myriad of cell signal transduction pathways and modulation of ion channel homeostasis. Examples of monoaminergic neurotransmitter receptors include the dopamine receptors: D1, D2, D3, D4 and D5; the serotonin (5-HT) receptors: 5-HT1, 5-HT2, 5-HT3, 5-HT4, 5-HT5, 5-HT6 and 5-HT7; the adrenergic receptors, i.e. the the noradrenaline receptors: alpha1, alpha 2, beta 1, beta2 and beta3 and the Acetylcholine receptors: M1, M2, M3, M4, NM and NN.

At least one of the monoaminergic neurotransmitter receptors in need of modulation is a 5-hydroxytryptamine receptor. The term "5-hydroxytryptamine receptor" (also 5-HT receptor" or "serotonin receptor") refers to a special group of G protein-coupled receptors (GPCRs) and ligand-gated ion channels (LGICs) found in the central and peripheral nervous systems. They mediate both excitatory and inhibitory neurotransmission. The serotonin receptors are activated by the neurotransmitter serotonin, which acts as their natural ligand. The serotonin receptors modulate the release of neurotransmitters, such as glutamate, GABA, dopamine, epinephrine / norepinephrine, and acetylcholine, as well as many hormones, including oxytocin, prolactin, vasopressin, cortisol, corticotropin, and substance P, among others. The serotonin receptors influence various biological and neurological processes or diseases such as aggression, anxiety, appetite, cognition, dementia, depression, learning, impulsive behavior, memory, mood, nausea, sleep, and thermoregulation. Thus, herein is disclosed the use of a compound with Formula I for the treatment and/or prevention of at least one of said biological and neurological processes or diseases when associated with a need for modulation of at least one 5-hydroxytryptamine receptor (5-HT receptor) influencing said biological and neurological processes or diseases.

The compounds of Formula I act as partial agonists on the monoaminergic neurotransmitter receptors. Partial agonists also bind and activate a given receptor, but have only partial efficacy at the receptor relative to a full agonist. Partial agonists can act as a competitive antagonist in the presence of a full agonist, as it competes with the full agonist for receptor occupancy, thereby producing a net decrease in the receptor activation as compared to that observed with the full agonist alone. The endogenous agonist for the 5-hydroxytryptamine receptor (5-HT receptor) is serotonin which is naturally produced by the host and binds to and activates this receptor.

At least one of the 5-hydroxytryptamine receptors (5-HT receptor) of the monoaminergic neurotransmitter receptors in need of modulation is of the group consisting of the 5HT₁, 5HT₂, 5HT₃, 5HT₄, 5HT₅, 5HT₆, and 5HT₇ receptors, i.e. more specifically one or more of the 5HT_{1A}, 5HT_{1B}, 5HT_{1D}, 5HT₁ₑ, 5HT_{1F}, 5HT_{2A}, 5HT_{2B}, 5HT_{2C}, 5HT₃, 5HT₄, 5HT_{5A}, 5HT₆, and 5HT₇ receptors. More specifically especially the 5-HT2 receptors, i.e. the 5HT_{2A}, 5HT_{2B}, 5HT_{2C} receptors are advantageously modulated in the present invention. It should be noted that the use of a compound of Formula I may include the modulation of more than one 5-HT receptor, i.e. two, three, four, five, six, seven, eight, nine, ten, eleven, twelve or more of the 5-hydroxytryptamine receptors listed above may be modulated simultaneously. It is also contemplated that all of the monoaminergic neurotransmitter receptor in need of modulation for a particular disease is of the 5-hydroxytryptamine receptor type only. However, also in such situations more than one 5-HT receptor as listed above may be modulated simultaneously.

Some diseases associated with a need for modulation of at least one 5-hydroxytryptamine receptor (5-HT receptor), may require the additional modulation of an additional type of monoaminergic neurotransmitter receptor, such as e.g. the dopamine receptors. When used herein the term "dopamine receptor" refers to a class of metabotropic G protein-coupled receptors that are prominent in the vertebrate central nervous system (CNS). The neurotransmitter dopamine is the primary endogenous ligand for dopamine receptors. Dopamine receptors are implicated in many neurological processes, including motivation, pleasure, cognition, memory, learning, and fine motor control, as well as modulation of neuroendocrine signaling. There are at least five subtypes of dopamine receptors, D₁, D₂, D₃, D₄, and D₅, all of which are included within the scope of the present invention. The D₁ and D₅ receptors are members of the D₁-like family of dopamine receptors, whereas the D₂, D₃ and D₄ receptors are members of the D₂-like family. The compounds of Formula I act as partial agonists on the dopamine receptors.

This at least one disease associated with a need for modulation of one or more monoaminergic neurotransmitter receptors, wherein at least one of the monoaminergic neurotransmitter receptors is a 5-HT receptor may be selected from the group consisting of depression, dementia, cognitive dysfunctions, aggressive behavior, impulsive behavior, obsessive-compulsive disorders (OCD) and anxiety disorders.

As used herein the expression "depression" is meant to include any mental disorder characterized by an all-encompassing low mood accompanied by low self-esteem, and by loss of interest or pleasure in normally enjoyable activities. Included in the expression are recurrent depressive disorders, clinical depression, major depression, unipolar depression, or unipolar disorders.

When used herein, the expression "dementia" refers to a serious loss of cognitive ability in a previously unimpaired person, beyond what might be expected from normal aging. It may be static, the result of a unique global brain injury, or progressive, resulting in longterm decline due to damage or disease in the body. Some of the most common forms of dementia are: Alzheimer's disease, vascular dementia, frontotemporal dementia, semantic dementia and dementia with Lewy bodies.

For example, in Parkinson's disease both depression and dementia are common and only partially respond to treatment with dopaminergic drugs. In Parkinson's disease a loss of serotonergic neurons in the brain, in addition to dopaminergic and noradrenergic neurons, is well recognized. Whereas serotonergic drugs, especially selective serotonin reuptake inhibitors (SSRIs) are widely and successfully used in various forms of depression, their efficacy in depressed Parkinson patients has not been clearly demonstrated. This could be due to the loss of serotonergic neurons in the brains of Parkinson patients, and thus of the targets for SSRIs, that is, the dopamine reuptake transporters. It is therefore proposed that 5-HT-receptor agonists such as those of the present invention will prove useful in the treatment of depression of various kinds, and especially in cases where substantial losses of serotonin neurons have occurred, such as in Parkinson patients. Nearly full 5-HT2-receptor agonists, e g LSD and DOI, are admittedly strong activators of various mental functions but are generally not suitable as therapeutic agents in view of their hallucinogenic and psychotogenic activities. On the other hand, a partial serotonin agonist which has been shown to lack such side effects even in high dosage, for example (-)-OSU6162, might well prove useful in this context. Thus, by compensating for the loss of both dopamine and serotonin functions occurring in Parkinson's disease such a compound should prove useful to alleviate not only motor but also some of the at least equally important mental dysfunctions occurring in this disorder, including depression and dementia.

A reduction of the serotonin level in the brains of patients with dementia of the Alzheimer type has been known for a long time. Reductions of acetylcholine (as indicated by choline acetylase), norepinephrine and dopamine levels have also been demonstrated. Treatment with cholinergic drugs (as acetylcholine esterase inhibitors) has been found to alleviate dementia in this disorder, although there is much room for additional improvement. It has been suggested that a mixture of cholinergic, noradrenergic, dopaminergic and serotonergic agonists could lead to better improvement than each agonist given separately. However, the availability of suitable serotonergic agonists has so far been limited. It is therefore proposed that the compounds of the present invention will prove useful in the treatment of dementia and cognitive dysfunctios of different types, such as in Alzheimer's and Parkinson's disease, or in chronic alcoholism (where changes similar to those found in Alzheimer's disease have been found, either when given alone or in combination with other cognitive enhancers.

Regarding the role of serotonin deficiency for the pathophysiology of dementia, a statistically significant negative correlation between the level of serotonin in the frontal cerebral cortex, measured post mortem, and the rate of cognitive decline per year preceding death has been found.

Moreover, strong support for a physiological role of 5-HT2A receptors in working memory has been reported. It was found that 5-HT2A receptor stimulation is facilitatory for the mnemonic process occurring in prefrontal macaque pyramidal cells participating in spatial working memory. This facilitatory effect could be demonstrated after iontophoretic application of 5-HT or its alpha-methylated derivative on to the cells, and the effect could be antagonized by the selective 5-HT2A antagonist M100907.

As used herein, the expression "cognitive dysfunctions" refers to a state of unusually poor mental function, associated with confusion, forgetfulness and difficulty concentrating. A number of medical or psychiatric conditions and treatments can cause such symptoms, including Alzheimer's disease, Parkinson's disease and chronic alcoholism, heavy metal poisoning (in particular mercury poisoning), menopause, fibromyalgia, mood disorders, ADHD and sleep disorders.

When used herein the expression "aggressive behavior" refers to a behavior between members of the same species that is intended to cause humiliation, pain, or harm. Furthermore, when used herein the expression "impulsive behavior" refers to a failure to resist an impulsive act or behaviour that may be harmful to self or others. An impulsive behaviour or act is considered to be one that is not premeditated or not considered in advance and one over which the individual has little or no control. Impulsive behavior may include the disorders Trichotillomania, Intermittent Explosive Disorder, Pathological Gambling,
Kleptomaniahttp://www.forensicpsychiatry.ca/forensic/Criminology/impulse/gambling.htm and/or Pyromania. Considerable evidence supports the view that aggressive behavior as well as impulsivity is promoted by serotonin hypofunction alone, but especially by the simultaneous occurrence of dopamine hyperfunction and serotonin hypofunction. The compounds of the present invention can thus be predicted to be useful in the prevention and treatment of aggressive behaviour, given their ability to counteract both these aberrations.

As used herein the term "Obsessive-compulsive disorder (OCD)" refers to an anxiety disorder characterized by intrusive thoughts that produce uneasiness, apprehension, fear, or worry, by repetitive behaviors aimed at reducing the associated anxiety, or by a combination of such obsessions and compulsions. Symptoms of the disorder include excessive washing or cleaning; repeated checking; extreme hoarding; preoccupation with sexual, violent or religious thoughts; aversion to particular numbers; and nervous rituals, such as opening and closing a door a certain number of times before entering or leaving a room.

Several reports have described beneficial effects of the 5-HT2A(C) receptor agonists LSD, mescaline, psilocin, psilocybin and peyote cactus in OCD. Also supporting the importance of 5-HT2A(C) receptors in the context of OCD is the growing number of reports describing a propensity of clozapine, a 5-HT2A/C receptor antagonist, to produce, unmask or exacerbate OCD symptoms in schizophrenic subjects. In addition, there have been reports of unmasking or worsening effects of risperidone, a 5-HT2A/DA D2 receptor antagonist, with respect to OCD symptoms in psychosis. Similar effects have subsequently been reported with several other second generation antipsychotics which display a higher relative 5-HT2 vs DA D2 receptor blockade than the classical neuroleptics. These studies underline the putative role of 5-HT2A receptors in OCD. Given the data summarized above partial HT2A(C) receptor agonists should prove beneficial in the treatment of OCD, provided that are devoid of hallucinogenic properties but still retain an intrinsic activity sufficient to alleviate OCD symptoms. (-)-OSU6162 seems to fulfill these criteria since it has been found not to be hallucinogenic even in high dosage in spite of a significant degree of intrinsic activity, as evidenced by the in-vitro and in-vivo data presented in the present invention.

Regarding other anxiety disorders, e g panic disorder, posttraumatic stress disorders, agoraphobia, generalized anxiety disorder, and premenstrual tension, they are likely to respond to treatment with the compounds of the present invention, given their responsiveness to SSRIs and other drugs capable of supporting insufficient serotonergic functions.

The following diseases associated with a need for modulation of at least one 5-hydroxytryptamine receptor (5-HT receptor), may require the modulation of an additional type of monoaminergic neurotransmitter receptor, such as the dopamine receptors. Neurological and psychiatric disorders may be characterized by a dysfunction of the dopamine system as well as the serotonin system. The neurological and psychiatric disorders may be selected from the group consisting of Parkinson's disease in early stages; restless legs; akathisia; dystonias; mental fatigue associated with high age, stroke, postencephalitic or posttraumatic conditions; attention-deficit disorders (ADD and ADHD); autism spectrum disorders; lapses of consciousness including narcolepsy, petit mal epilepsy and syncope; sleeping disorders including hypersomnia, sleep apnea, and attacks of sleep induced by dopamine receptor agonists, dopamine hypofunction induced by antipsychotic drugs. Tourette's syndrome, and chronic fatigue syndrome (CFS).

The compound used in the present invention may be 3-[3-(methylsulfonyl)phenyl]-1-propylpiperidine or pharmaceutically acceptable salts thereof. Also disclosed herein are (3S)-3-[3-(methylsulfonyl)phenyl]-1-propylpiperidine, (3R)-3-[3-(methylsulfonyl)phenyl]-1-propylpiperidine or pharmaceutically acceptable salts thereof.

Also disclosed herein are 3-(3-cyanophenyl)-1-propylpiperidine, (3S)- 3-(3-cyanophenyl)-1-propylpiperidine, (3R)- 3-(3-cyanophenyl)-1-propylpiperidine or pharmaceutically acceptable salts thereof.

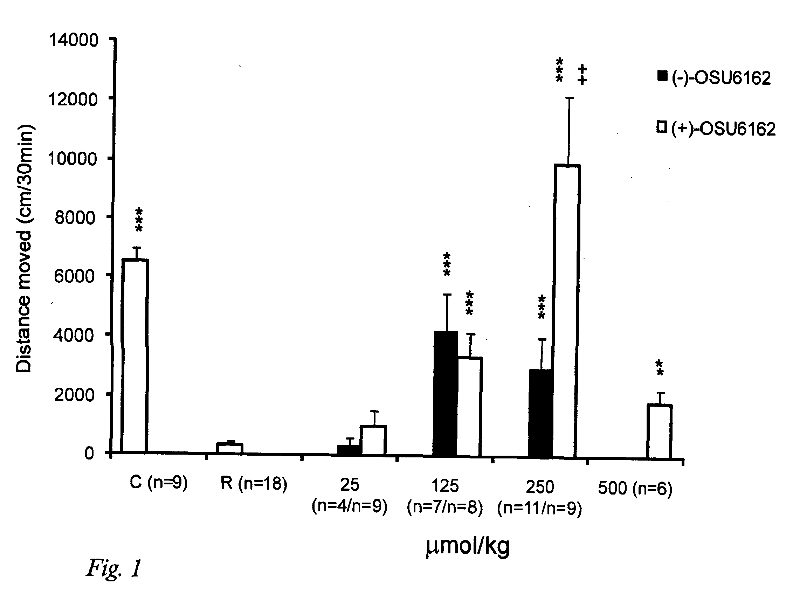

The significantly stronger serotonin-dependent psychomotor stimulation by the (+)-compared to the (-)-form of OSU6162 in monoamine-depleted mice (see e.g. Figure 1, below), suggests that the (+)-forms i.e. (3R)-3-[3-(methylsulfonyl)phenyl]-1-propylpiperidine and/or (3R)- 3-(3-cyanophenyl)-1-propylpiperidine or pharmaceutically acceptable salts thereof will have a stronger therapeutic effect than the (-)-form on dementia and depression, especially in neurodegenerative conditions characterized by loss of serotonin, such as Parkinson's disease and dementia."

The compound of Formula I may be formulated for appropriate administration to a subject.

### Oral/buccal/sublingual

For oral, buccal or sublingual administration, the compounds of the present invention may be combined with various excipients. Solid pharmaceutical preparations for oral administration often include binding agents (for example syrups and sugars, acacia, gelatin, sorbitol, tragacanth, polyvinylpyrrolidone, sodium lauryl sulphate, pregelatinized maize starch, hydroxypropyl methylcellulose, lactose, starches, modified starches, gum acacia, gum tragacanth, guar gum, pectin, wax binders, microcrystalline cellulose, methylcellulose, carboxymethylcellulose, hydroxypropyl methylcellulose, hydroxyethyl cellulose, hydroxypropyl cellulose, copolyvidone and sodium alginate), disintegrants (such as starch and preferably corn, potato or tapioca starch, alginic acid and certain complex silicates, polyvinylpyrrolidone, sucrose, gelatin, acacia, sodium starch glycollate, microcrystalline cellulose, crosscarmellose sodium, crospovidone, hydroxypropyl methylcellulose and hydroxypropyl cellulose), lubricating agents (such as magnesium stearate, sodium lauryl sulfate, talc, silica polyethylene glycol waxes, stearic acid, palmitic acid, calcium stearate, carnuba wax, hydrogenated vegetable oils, mineral oils, polyethylene glycols and sodium stearyl fumarate) and fillers (including high molecular weight polyethylene glycols, lactose, sugar, calcium phosphate, sorbitol, glycine magnesium stearate, starch, glucose, lactose, sucrose, rice flour, chalk, gelatin, microcrystalline cellulose, calcium sulphate, xylitol and lactitol). Such preparations may also include preservative agents and anti-oxidants.

Liquid compositions for oral administration may be in the form of, for example, emulsions, syrups, or elixirs, or may be presented as a dry product for reconstitution with water or other suitable vehicle before use. Such liquid compositions may contain conventional additives such as suspending agents (e.g. sorbitol, syrup, methyl cellulose, hydrogenated edible fats, gelatin, hydroxyalkylcelluloses, carboxymethylcellulose, aluminium stearate gel, hydrogenated edible fats) emulsifying agents (e.g. lecithin, sorbitan monooleate, or acacia), aqueous or non-aqueous vehicles (including edible oils, e.g. almond oil, fractionated coconut oil) oily esters (for example esters of glycerine, propylene glycol, polyethylene glycol or ethyl alcohol), glycerine, water or normal saline; preservatives (e.g. methyl or propyl p-hydroxybenzoate or sorbic acid) and conventional flavouring, preservative, sweetening or colouring agents. Diluents such as water, ethanol, propylene glycol, glycerin and combinations thereof may also be included.
Other suitable fillers, binders, disintegrants, lubricants and additional excipients are well known to a person skilled in the art.

### Vaginal/rectal

The compounds according to the present the invention may also be formulated in a form suitable for rectal or vaginal use. For rectal or vaginal administration, the formulation may be prepared in the form of a suppository. Suppository formulations may be prepared by mixing the active ingredient with a suitable non-irritating excipient which is solid at ordinary temperatures but liquid at the body temperature and will therefore melt in the body to release the drug. Such suppositories contain the active substance mixed with a neutral fat base (for example, cocoa butter or polyethylene glycols), or in the form of a gelatin capsule which contains the active substance in a mixture with a vegetable oil, paraffin oil or other suitable vehicle. For rectal administration, enemas can be formulated, in which the dosage units take the form of a ready-made micro enema; or dry micro enema formulation to be reconstituted in a suitable solvent prior to administration.

### Nasal/inhalation

For intranasal administration or administration by inhalation, the compounds of the present invention may be delivered in the form of a solution, dry powder or suspension. Administration may take place via a pump spray container that is squeezed or pumped by the patient or through an aerosol spray presentation from a pressurized container or a nebulizer, with the use of a suitable propellant, e.g., dichlorodifluoromethane, trichlorofluoromethane, dichlorotetrafluoroethane, carbon dioxide or other suitable gas. The compounds of the invention may also be administered via a dry powder inhaler, either as a finely divided powder in combination with a carrier substance (e.g. a saccharide) or as microspheres. The inhaler, pump spray or aerosol spray may be single or multi dose. The dosage may be controlled through a valve which delivers a measured amount of active compound.

### Topical/skin patches

It is possible to administer the compounds of the present invention topically. This may be done by way of creams, jellies, gels, pastes, patches, aqueous or oily solutions or suspensions, ointments and the like. Such formulations are well known to those skilled in the art.

The compounds of the invention may be administered transdermally by means of a skin-patch formulation. In an example, a compound of the invention is dispersed in an adhesive which adheres to the skin, thereby permitting the compound to diffuse from the adhesive through the skin for delivery to the patient. For a steady rate of percutaneous absorption, pressure sensitive adhesives such as natural rubber or silicone can be used.

### Parenteral (I.V and I.M)

The compounds of the present invention may be formulated in an injectable form in an aqueous or non-aqueous solution, suspension or emulsion in a pharmaceutically acceptable liquid, e.g. sterile water, 1,3-butanediol or a parenterally acceptable oil or a mixture of liquids.

The liquid may contain bacteriostatic agents, anti-oxidants or other preservatives, buffers, solutes, thickening agents, wetting agents, suspending agents or other pharmaceutically acceptable additives. It is common that the liquid is isotonic with blood (e.g. through the addition of salts or glucose), and usually has a pH >8. The liquid is dispensed into unit doses in the form of ampoules, disposable injection devices or vials. Alternatively, the formulation is in the form of a concentrate or a dry preparation which can be reconstituted before use to prepare an injectable formulation.

### Controlled/delayed/prolonged release formulation

The compounds of the invention may also be administered in a controlled release formulation. The compounds are released at the required rate to maintain constant pharmacological activity for a desirable period of time. Such dosage forms provide a supply of a drug to the body during a predetermined period of time and thus maintain drug levels in the therapeutic range for longer periods of time than conventional non-controlled formulations. The compounds may also be formulated in controlled release formulations in which release of the active compound is targeted. For example, release of the compound may be limited to a specific region of the digestive system through the pH sensitivity of the formulation. Such formulations are well known to persons skilled in the art.

### Liposomes

The active compounds may be administered in the form of liposome delivery systems, such as small unilamellar vesicles, large unilamellar vesicles and multilamellar vesicles. Liposomes can be formed from a variety of phospholipids, such as cholesterol, stearylamine or phosphatidylcholines.

### Examples

In the following examples the invention will be described in more detail. However, the described embodiments mentioned below are only given as examples and should not be limiting to the present invention. Other solutions, uses, objectives, and functions within the scope of the invention as claimed in the below described patent claims should be apparent for the person skilled in the art.

### In vivo experiments

### Materials and Methods

### Animals

Male NMRI mice (Charles-River, Germany) weighing 20-35 g at the time of testing were used in the different experiments. The mice were housed in groups of eight in ventilated, wood wool-enriched, Macrolon type III cages for at least one week before the experiments were carried out. The mice were maintained under a 12-h light/dark cycle with lights on at 6:00 AM; the experiments were carried out during the light phase.

Motor activity experiments in both active and habituated rats were performed on male Sprague-Dawley rats (Charles River, Germany) weighing 280-320 g. Prior to testing, rats were housed in groups of four or five in ventilated Macrolon type IV cages for approximately one week. Habituated animals were maintained under a 12-h light/dark cycle with lights on at 6:00 AM; the experiments were carried out during the light phase. Active animals were maintained under a reversed daylight cycle (12-h light/dark cycle with lights off at 6:00 AM); the experiments were carried out during the dark phase All animals were maintained in a temperature of 20°C and had free access to water and food pellets. The experiments were approved by the Göteborg Ethic Committee for Animal Experimentation.

### Drugs

(-)-OSU6162 hydrochloride (MW=317.9), under the synonym PNU-96391A, was a gift from Pfizer, (+)-OSU6162 hydrochloride (MW=317.9), under the synonym R-PNU-96391, was synthesized by Syntagon, Sweden. Haloperidol (MW=375.9), raclopride tartrate salt (S(-)-raclopride (+)-tartrate salt, MW=497.3), reserpine (Crystalline; MW= 608.7) and SCH23390 (R(+)-SCH-23390 hydrochloride, MW=324.2) were purchased from Sigma-Aldrich Sweden. SCH39166 hydrobromide (MW=394.7) was purchased from Tocris Bioscience. M100907 ((+)-(R)-1-[1-[2-(4-fluorophenyl)ethyl]piperidin-4-yl]-1-(2,3-dimethoxyphenyl)methanol; MDL100,907; MW= 373.5) was a gift from Aventis. DOI [(±)-1-(2,5-dimethoxy-4-iodophenyl)-2-aminopropane hydrochloride] was purchased from Sigma/RBI.

(-)-OSU6162 and (+)-OSU6162 were dissolved in physiological saline (0.9%) and administered intraperitoneally (ip) in mice and subcutaneously (sc) in rats. Haloperidol, SCH39166 and M100907 were dissolved in a minimum amount of acetic acid and diluted with 5.5% glucose. The solution was adjusted to pH 5-7 with sodium bicarbonate. Haloperidol was given ip in mice and sc in rats. SCH39166 was given sc in mice and rats. M100907 was given ip in mice and rats. Reserpine was dissolved in a minimum amount of acetic acid and diluted with 5.5% glucose, and injected ip. Raclopride and SCH23390 were dissolved in physiological saline and injected sc. DOI was dissolved in physiological saline and injected ip in mice. Reserpine was given in a volume of 20 ml/kg in mice. All other substances were injected in a volume of 10 ml/kg in mice and 5 ml/kg in rats. The pH was measured in all solutions and adjusted to pH 5-7 with sodium bicarbonate if necessary (with the exception of reserpine). Control groups received appropriate vehicle treatment.

### Video tracking experiments in mice

### Equipment

Eight black Plexiglas arenas (I, w, h: 46×33×35 cm), indirectly illuminated to avoid reflections and shadows, were used. To obtain indirect lightning, the light fittings were placed below the upper edge of the Plexiglas arena. This produced an illumination at the floors of the arenas of approximately 5-7 lux. The floors of the arenas were covered with grey gravel previously exposed to other mice. The arenas were filmed from above with two monochrome video cameras, one camera per setting of four arenas, connected to a PC and recorded with Noldus MPEG Recorder 2.0.

After completion of the experiment recordings were analyzed with the video-tracking program EthoVision 3.1 Color Pro from Noldus Information Technology, Wageningen, The Netherlands. Animal movements were automatically tracked by the program and the distance moved by the animal was calculated in two ways (see below). Tracking was performed at a sampling frequency of 12.5 samples per second.

### Behavioral testing of monoamine-depleted mice

Animals were injected ip with reserpine (10 mg/kg, 20 ml/kg) 20 h prior to video recording. After reserpine treatment the animals were allowed to settle in Macrolon type III cages containing a small amount of sawdust, food and water. The cages were then placed on a heating mat and illuminated with a dim red light to keep the animals warm. Control animals were given 5.5% glucose (20 ml/kg, ip) 20 h prior to video recording and were housed in separate Macrolon type III cages under the same conditions, except for the heating.

On the day of the experiment animals were weighed, marked and in the antagonist experiments the animals were injected with antagonist prior to agonist and activity recording. Haloperidol and raclopride were injected 1 h prior to filming. M100907 was injected 30 minutes (in one experiment with (-)-OSU6162) or 1 h prior to video recording. (-)-OSU6162 and (+)-OSU6162 were injected just prior to recording and the animals were placed in the arenas, one animal in each arena, and videotaped for 60 minutes.

### Behavioral testing of drug naïve mice

On the day of the experiment animals were weighed, marked and injected ip with (-)-OSU6162 or (+)-OSU6162 15 minutes prior to video recording. Animals were placed in the arenas, one animal in each arena, and videotaped for 60 minutes.

### Procedure head twitch experiments

The animals used in the head twitch experiments were individually placed in Macrolon type II cages and allowed to habituate to these test cages during 30 minutes before the video registration of behavior began. DOI (1 mg/kg) or various doses of (-)-OSU6162 or (+)-OSU6162 were given 10 minutes before the video recording started. The animals were videotaped for 10 minutes; the behavior was scored during the first 5 minutes. The number of head twitches was counted from the videotapes by a person blind to the treatment.

### Data and statistical analyses

Distance moved was calculated in two ways resulting in the variables DM or DM1.5. DM is the total distance moved by the animal, calculated from all horizontal movements of the animal. DM1.5 is distance calculated using a distance filter of 1.5 cm, meaning that the animal has to move a distance of ≥1.5 cm before distance registration occurs. Data are expressed as means and standard error of the mean (S.E.M.). In several cases data from two or more experiments have been pooled. "C" in figures denotes control group. Group comparisons were performed with Mann-Whitney U test. All statistical tests were two sided and p<0.05 was considered statistically significant.

### Motor activity experiments in habituated and active rats

### Behavioral testing of habituated rats

These experiments were designed to resemble those of Sonesson et al (Sonesson C, Lin CH, Hansson L, Waters N, Svensson K, Carlsson A, Smith MW, Wikström H (1994) Substituted (S)-phenylpiperidines and rigid congeners as preferential dopamine autoreceptor antagonists: synthesis and structure-activity relationships. J Med Chem 37(17):2735-2753).. Rats were housed in a normal daylight cycle and the experiments were carried out during daytime. Animals were introduced into sound attenuated, illuminated activity boxes (l/w/h: 40×40×20 cm) and were allowed to habituate in the new environment for 65 minutes. During the following 5 minutes test drugs were injected, after which the rats were returned to the boxes for another 60 minutes. Five by five rows of photocell beams at floor level allowed a computer based-system to register horizontal activity. Motor activity is presented as accumulated number of unrepeated beam breaks, i.e. several consecutive breakings of one beam is counted as one beam break. The software was set to register behavior from the rats' first introduction into the arena until it was finally removed from the box, i.e. for 110 or 130 minutes.

### Behavioral testing of active rats

This method has been described previously by Rung et al. (Rung JP, Rung E, Helgeson L, Johansson AM, Svensson K, Carlsson A, Carlsson ML (2008) Effects of (-)-OSU6162 and ACR16 on motor activity in rats, indicating a unique mechanism of dopaminergic stabilization. J Neural Transm 115(6):899-908). The rats were housed in reverse daylight cycle and all handling of the animals was performed in dim light. Test drugs were administered s.c. 30 minutes prior to registration of behavior. Single rats were introduced into rectangular arenas (l/w/h: 150×100×40 cm) illuminated indirectly by one infrared (IR) lamp (Neocom, South Korea). The rats' movements were recorded to digital (MPEG2) video files using an IR sensitive video camera (Panasonic WV-CPR480, lens: Panasonic LA-408C3) connected to a PC equipped with a MPEG-encoder (MVR1000SX, Canopus Co.). The video files were then analyzed with the video tracking software EthoVision 3.1 Color Pro (Noldus Information Technology, Wageningen, The Netherlands) using a sample frequency of 12.5 samples per second.

### Data and statistical analyses

Data are expressed as means and standard error of the mean (S.E.M.). In several cases data from two or more experiments have been pooled. Suspected outliers were evaluated one at the time with Grubbs' test for outliers (Grubbs FE (1969) Procedures for detecting outlying observations in samples. Technometrics 11(1):1-21). Group comparisons were performed with Mann-Whitney U-test. All statistical tests were two-sided and p<0.05 was considered statistically significant.

### Results

### Monoamine-depleted mice

The first series of experiments described below was performed in monoamine-depleted mice. Depletion of monoaminergic stores was accomplished by pretreatment with 10 mg/kg of reserpine injected intraperitoneally (i.p.) 20 h prior to the activity recording.

### Stimulatory effects of (-)-OSU6162 and (+)-OSU6162 in monoamine-depleted mice

It can be seen in Figure 1 that (-)-OSU6162 and (+)-OSU6162 each stimulated motor activity in mice pretreated with reserpine. Activity was recorded for 60 minutes in a video tracking setting. Plotted data (distance moved; DM 1.5.) are based on the first 30 minutes of the 60 minutes recording period. Shown are means and SEM. C= control animals. R= animals treated with only reserpine. All animals, except control animals, were injected with reserpine (10 mg/kg, ip) 20 h prior to activity recording. Control animals were injected with glucose solution (5.5%, ip) 20 h prior to activity recording and NaCl (0.9%, ip) just prior to activity recording. (-)-OSU6162 (25-250 µmol/kg ip) or (+)-OSU6162 (25-500 µmol/kg ip) was injected just prior to activity recording. Statistical comparisons were made by Mann-Whitney U-test; *: vs R group, +: vs (-)-OSU6162, 250 µmol/kg. **/++ p<0.01, *** p<0.001.

(-)-OSU6162 caused a statistically significant increase in motor activity at 125 and 250 µmol/kg. (+)-OSU6162 caused a statistically significant increase in motor activity at 125, 250 and 500 µmol/kg, with a peak response at 250 µmol/kg. At 250 µmol/kg the two enantiomers differed significantly (p < 0.01) from each other, (+)-OSU6162 causing a much larger effect on motor activity (Figure 1), and also a more long-lasting effect (not shown), than the (-)-enantiomer.

### Antagonizing the effects of (-)-OSU6162 and (+)-OSU6162 in monoamine-depleted mice

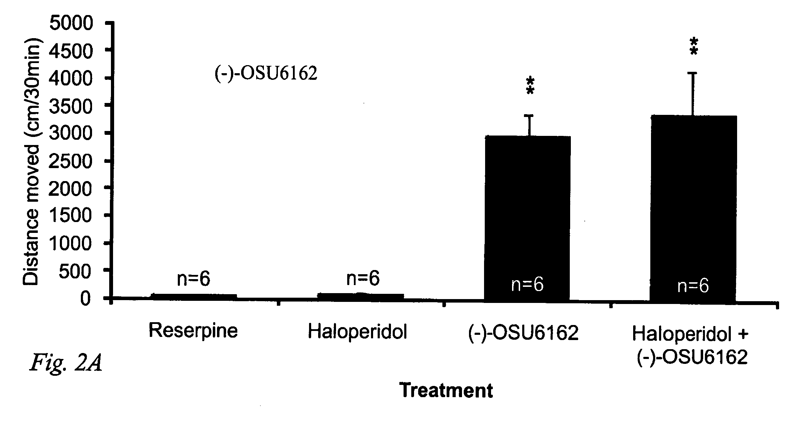

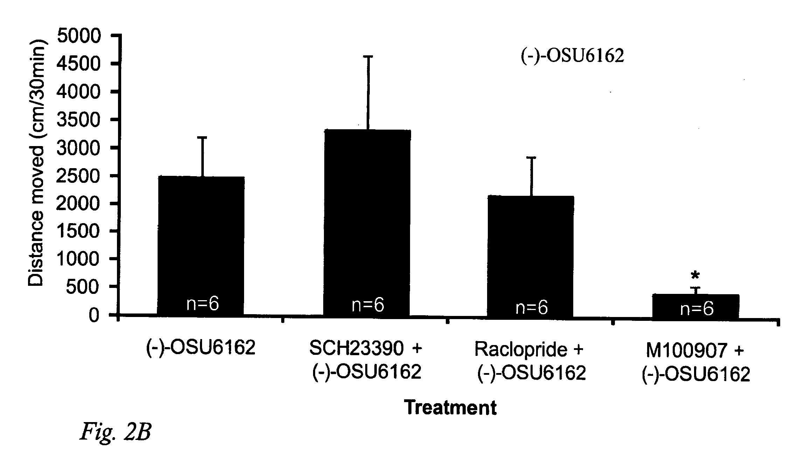

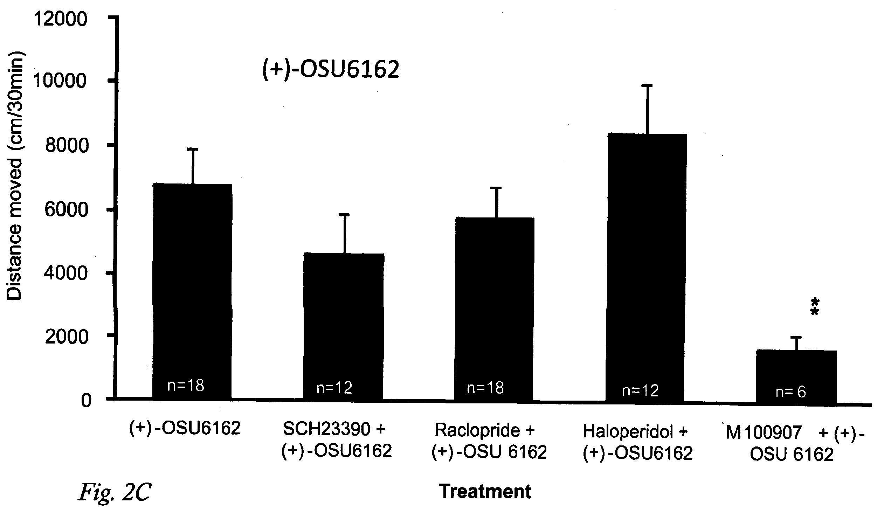

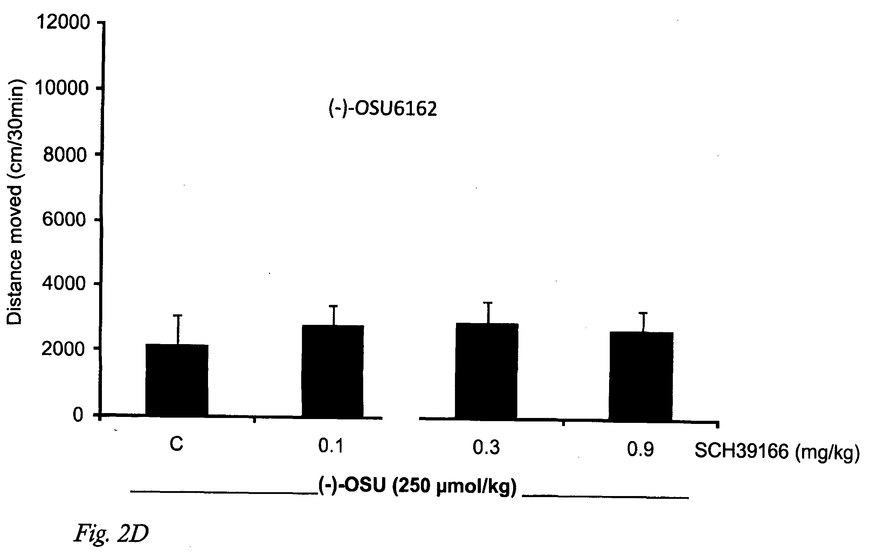

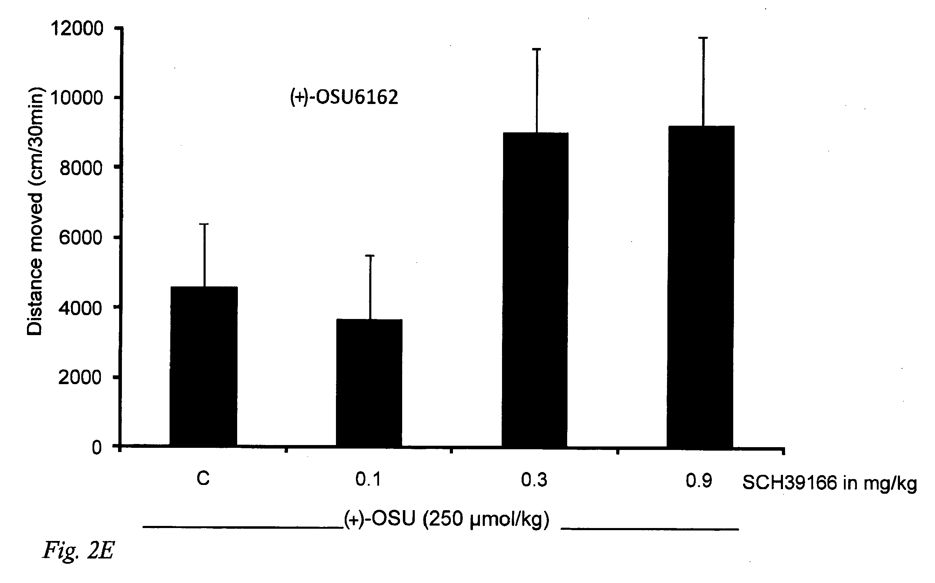

A series of experiments was carried out with the aim of identifying the receptors and mechanisms involved in the stimulatory effects of (-)-OSU6162 and (+)-OSU6162 on motor activity in reserpine-pretreated mice. A battery of monoaminergic receptor antagonists were tested with respect to their ability to counteract the stimulatory effects of (-)-OSU6162 and (+)-OSU6162(see figures 2-4). In all experiments activity was recorded for 60 minutes in a video tracking setting. Plotted data (DM 1.5) are based on the first 30 minutes of the 60 minutes recording period. Shown are means and SEM. All animals were injected with reserpine (10 mg/kg, ip) 20 h prior to activity recording.

Figure 2 a,b. Haloperidol (1 mg/kg, ip), raclopride (20 mg/kg, sc) and M100907 (1 mg/kg, ip) were given one hour prior to (-)-OSU6162 (250 µmol/kg, ip) and activity recording. SCH23390 (0.3 mg/kg, sc) was injected twice, one hour before and immediately before activity recording. (-)-OSU6162 was also injected just prior to activity recording. Statistical comparisons were made by Mann-Whitney U-test vs. a) the reserpine group and b) the (-)-OSU6162 group; * p<0.05, ** p<0.01. It is seen that the dopamine D2 receptor antagonist haloperidol (1 mg/kg) could not antagonize the stimulating effects of (-)-OSU6162 (250 µmol/kg) on motor activity in mice pretreated with reserpine (Figure 2a). This was also true for the dopamine D1 blocker SCH23390 (0.3 mg/kg) and the dopamine D2 blocker raclopride (20 mg/kg; Figure 2b). The selective 5-HT2A receptor antagonist M100907 (1 mg/kg), on the other hand, effectively antagonized the (-)-OSU6162-induced behavioral stimulation (Figure 2b).

Figure 2c. Raclopride (20 mg/kg, sc), haloperidol (1 mg/kg, ip) and M100907 (1 mg/kg, ip) were injected 1h prior to activity recording. SCH23390 (0.3 mg/kg, sc) was injected twice, once 1 h and once just prior to activity recording. (+)-OSU6162 (250 µmol/kg, ip) was injected immediately before activity recording started. Statistical comparisons were made by Mann-Whitney U-test: ** p<0.01 vs. the (+)-OSU6162 group.

The results from the antagonist experiments with (+)-OSU6162 are similar to those seen for (-)-OSU6162. None of the dopaminergic antagonists, i e SCH23390 (0.3 mg/kg), raclopride (20 mg/kg) or haloperidol (1 mg/kg), could antagonize the motor activity stimulating effects of (+)-OSU6162 (250 µmol/kg) in reserpine-pretreated mice whereas M100907 (1 mg/kg) effectively antagonized this response (Figure 2c).

Another D1 antagonist, SCH39166, presumed to be more selective for D1 receptors than SCH23390. SCH39166 (0.1, 0.3 or 0.9 mg/kg, sc) was given 20 minutes prior to (-)-OSU6162 or (+)-OSU6162 (both 250 µmol/kg, ip) and activity recording. The results are shown in Figure 2d and e (in d) n=6 and in e) n=5). It is can be seen that SCH39166 (0.1, 0.3, 0.9 mg/kg) did not significantly influence the motor activation caused by (-) or (+)-OSU6162 (250 µmol/kg). This is in accordance with the results from the SCH23390 experiments above (Figs. 2b and c).

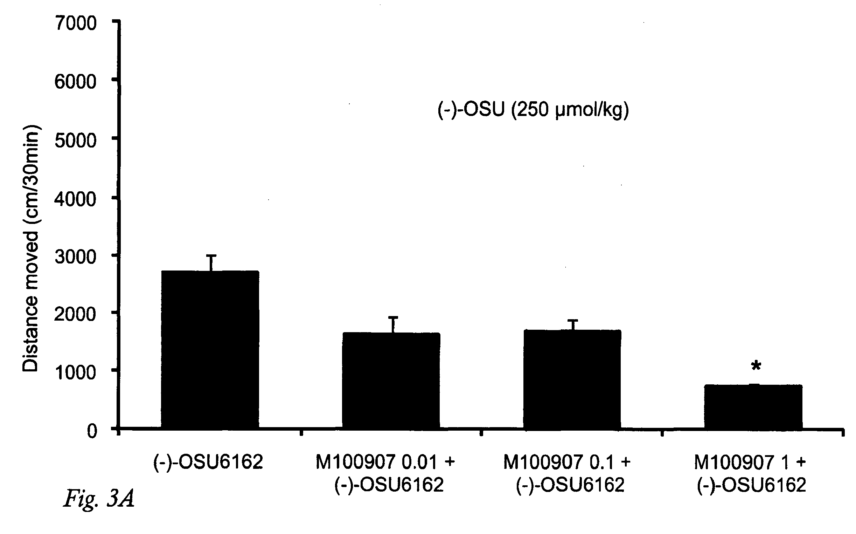

Figures 3a-c show dose response curves of the effects of M100907 on locomotor stimulation induced by the OSU6162 enantiomers. All animals, except for the Control group in c, were injected with reserpine (10 mg/kg, ip) 20 h prior to activity recording. (-)-OSU6162 and (+)-OSU6162 were injected immediately before the start of activity recording. In a) M100907 (0.01, 0.1 or 1 mg/kg) was injected ip 30 minutes prior to (-)-OSU6162 (250 µmol/kg, ip). In b) M100907 (0.01, 0.1 or 1 mg/kg) was injected ip 30 minutes prior to (-)-OSU6162 (125 µmol/kg). In c) M100907 (0.1 or 1 mg/kg) was injected ip 30 minutes prior to (+)-OSU6162 (125 µmol/kg). N= 2 for Control group; n=5 for Reserpine and M100907 1+OSU groups; n=6 for OSU and M100907 0.1+OSU groups. Statistical comparisons were made by Mann-Whitney U-test: * p<0.05, ** p<0.01 vs. the (-)/(+)-OSU6162 group.

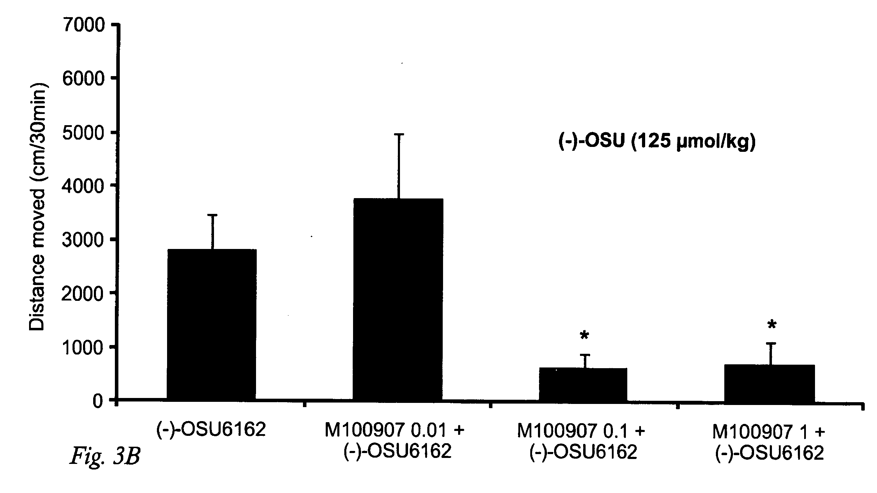

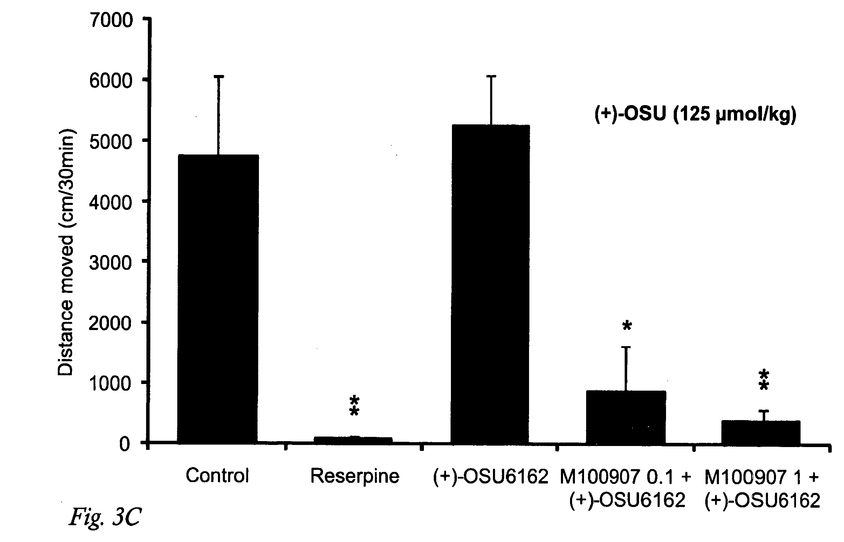

Figure 3a shows the effects of three doses (0.01, 0.1 and 1 mg/kg) of M100907 on the motor activity increase caused by 250 µmol/kg of (-)-OSU6162. Only the 1 mg/kg dose significantly decreased motor activity. Figures 3b and c show that 0.1 and 1 mg/kg of M100907 effectively reversed the locomotor stimulation induced by 125 µmol/kg of (-)-and (+)-OSU6162.

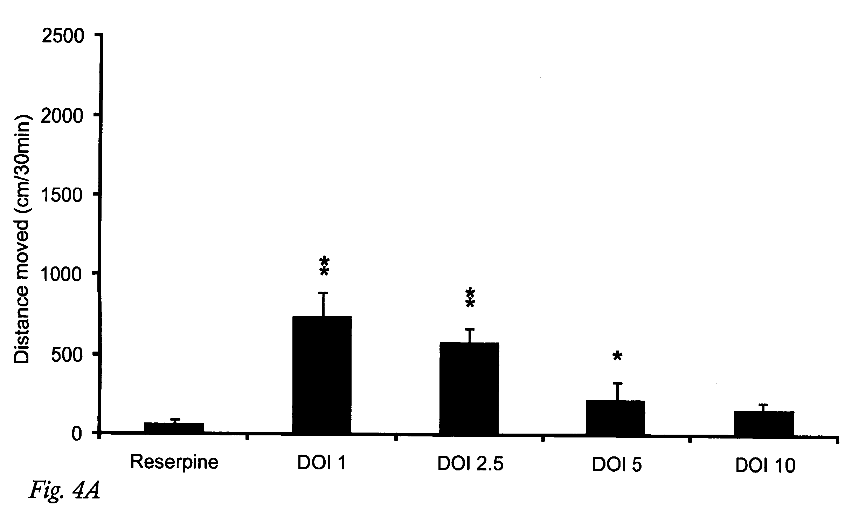

The effects of DOI on locomotion in reserpine-pretreated mice was tested (see Figures 4a-b: In a) DOI (1; 2.5; 5; 10 mg/kg) was injected ip just prior to activity recording. N=4 in group DOI10 and 5 in all other groups. Statistical comparisons were made by Mann-Whitney U-test: * p<0.05 , ** p<0.01 vs. the group receiving reserpine only. Figure 4a shows that also the 5-HT2 agonist DOI (1; 2.5; 5; 10 mg/kg) was able to induce locomotor stimulation in reserpine-pretreated mice, although the degree of stimulation was lower than in the case of the OSU6162 enantiomers, particularly the (+)-enantiomer.

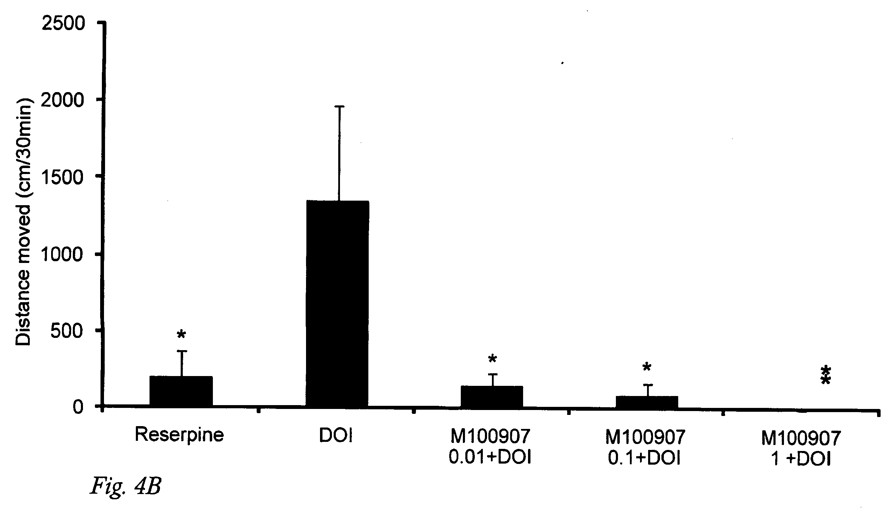

In b) the antagonizing effects of M100907 on the locomotor stimulation induced by DOI in reserpine-pretreated mice are shown. M100907 (0.01, 0.1, or 1 mg/kg) was injected ip 30 minutes prior to DOI (2.5 mg/kg), which was injected immediately before the activity recording started. N=4 in group 0.01+DOI and 5 in all other groups. Statistical comparisons were made by Mann-Whitney U-test: * p<0.05, ** p<0.01 vs. the DOI group. Figure 4b shows that M100907 was highly effective in antagonizing the locomotor stimulant effects of 2.5 mg/kg of DOI - all doses (0.01, 0.1 and 1 mg/kg) of M100907 had significant effects.

### Drug naive mice

The second series of experiments was performed in drug naive mice.

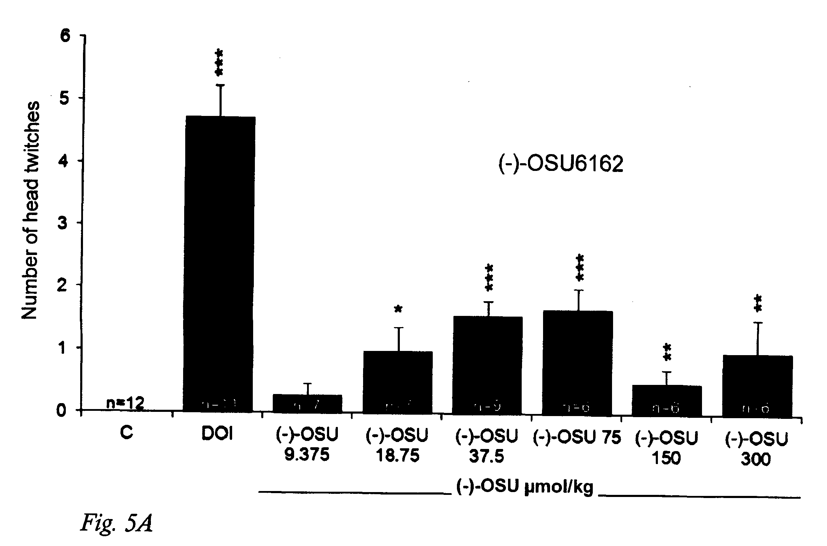

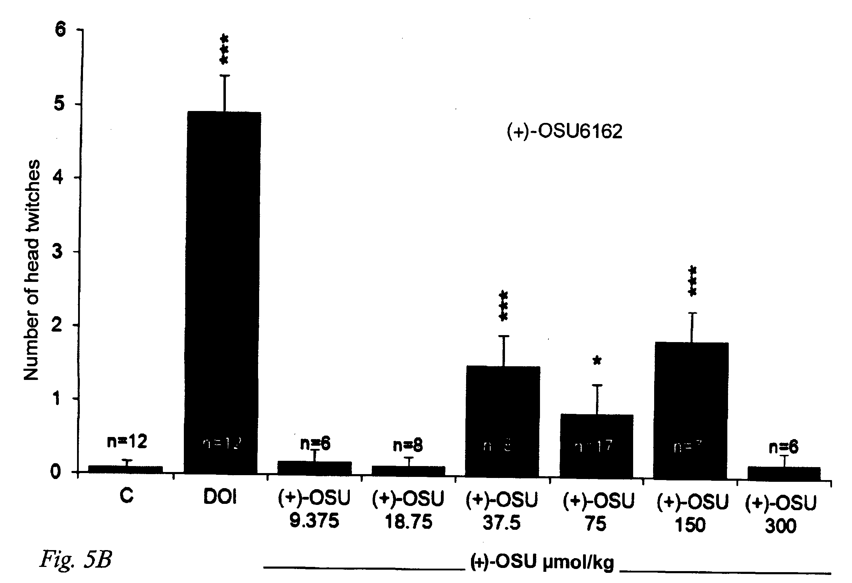

The purpose of the first experiment was to investigate whether (-)-OSU6162 and (+)-OSU6162 were able to produce head twitches in mice, a behavior typically produced by 5-HT2 agonists. (-)-OSU6162 and (+)-OSU6162 were compared to DOI in this regard (see figure 5).

In Figure 5, the number of head twitches produced during 5 minutes is shown. The mice were habituated to the test cages during 30 minutes before the video registration of behavior began. DOI (1 mg/kg ip), (-)-OSU6162 (a) or (+)-OSU6162 (b) (9.375; 18.75; 37.5; 75; 150; 300 µmol/kg ip) was administered 10 min before the registration of behavior started. Shown are means and SEM, n = 6-12. Statistical comparisons were made by Mann-Whitney U-test: *p < 0.05, **p < 0.01, ***p < 0.001 vs the control (C) group.

It can be concluded that DOI (1 mg/kg ip) induces head twitches in the mouse. Several of the tested doses of (-)-OSU6162 and (+)-OSU6162 (9.375; 18.75; 37.5; 75; 150; 300 µmol/kg) also produced head twitches but to a lesser degree than DOI.

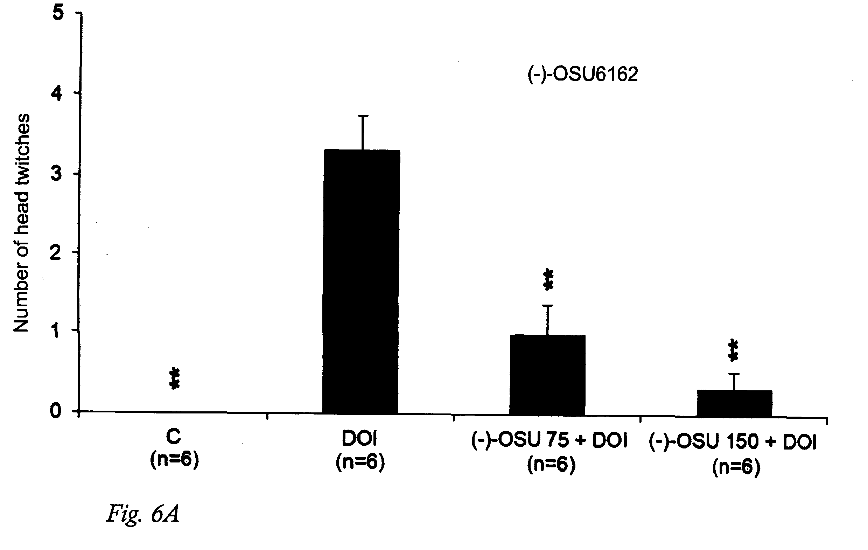

The purpose of the second experiment was to investigate whether the OSU6162 enantiomers would be able to counteract DOI-induced head twitches. Figure 6a shows the number of head twitches produced during 5 minutes. The mice were habituated to the test cages during 30 minutes before the video registration of behavior began. All compounds were administered 10 min before the registration of behavior started. DOI was given in dose of 1 mg/kg (ip) throughout. In a) the interaction between (-)-OSU6162 (75 or 150 µmol/kg ip) and DOI is shown. In b) the interaction between (+)-OSU6162 (75 or 150 µmol/kg ip) and DOI is shown. In c) the interaction between (-)- or (+)-OSU6162 (75 µmol/kg ip) and DOI is shown. Statistical comparisons were made by Mann-Whitney U-test: *p < 0.05, **p < 0.01, vs DOI. ++ p < 0.01 vs the group receiving (+)-OSU6162 + DOI.

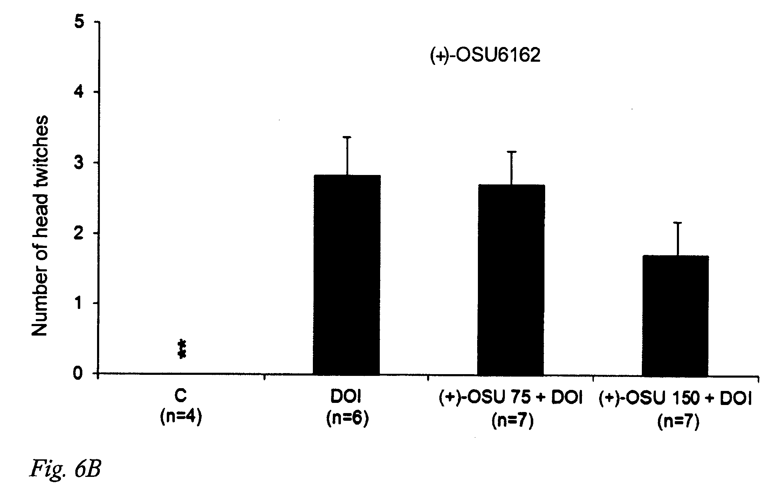

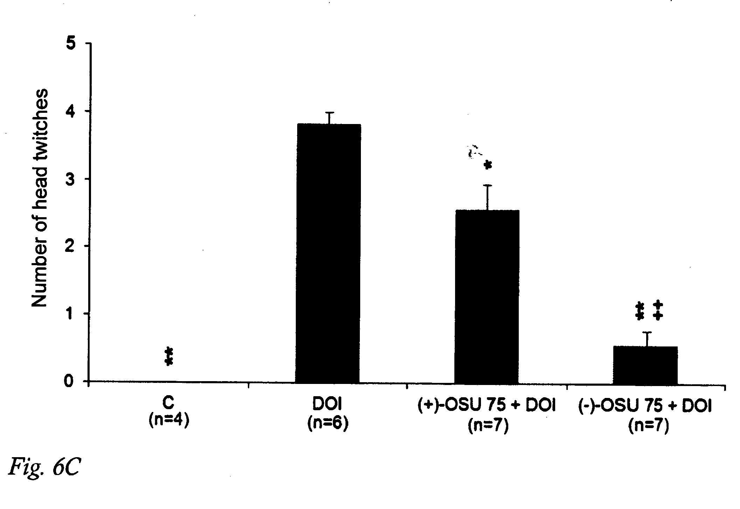

It is seen that 75 and 150 µmol/kg of (-)-OSU6162 significantly antagonized DOI-induced head twitches. Figure 6b shows that neither 75 nor 150 µmol/kg of (+)-OSU6162 significantly antagonized DOI-induced head twitches, although there was a tendency for the higher dose to do so. Figure 6c, finally, shows that 75 µmol/kg of both OSU6162 enantiomers counteracted DOI-induced head twitches, the (-)-enantiomer being considerably more effective than the (+)-enantiomer.

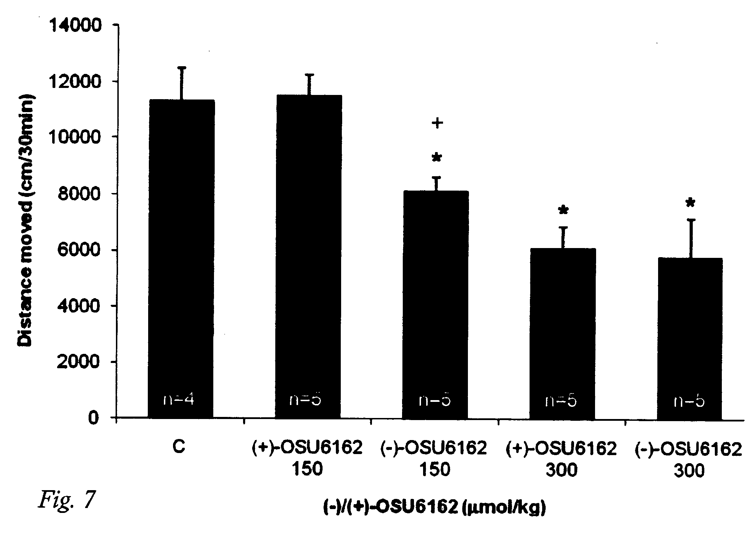

The purpose of the third experiment was to compare high doses of (-) and (+)-OSU6162 with respect to their effect on motor activity in drug naive mice (see figure 7). Data (DM) is plotted as means and SEM. C= control animals. N=4 in the C-group and 5 in all other groups. (-)-OSU6162, (+)-OSU6162 (150 or 300 µmol/kg) or NaCl (0.9%, Control animals) was injected ip 15 minutes prior to activity recording. Activity was recorded for 60 minutes in a video tracking setting. Plotted data are based on the first 30 minutes of the 60 minutes recording period. Statistical comparisons were made by Mann-Whitney U-test. * p<0.05 vs. the C group; + p<0.05 vs. (+)-OSU6162 150 µmol/kg. As evident from Figure 7 both enantiomers decreased motor activity, the (-)-form being more potent than the (+)-form.

### Rats

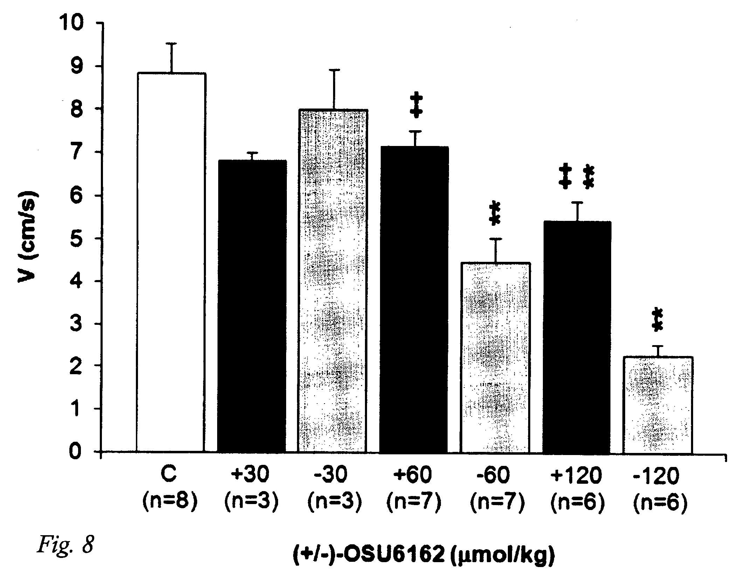

The third series of experiments was performed in drug naive rats (figure 8). Rats were injected sc with saline (C, 0.9%) or either test drug 30 minutes prior to video recording. (-)-OSU6162 and (+)-OSU6162 (30, 60, 120 µmol/kg) are denoted - and + respectively in the graph. Behavior was registered under infrared light in rectangular arenas (150×100×40 cm) by video tracking. Motor activity is shown as velocity. Shown are means and SEM. Statistical comparisons were made by Mann-Whitney U-test. ** p<0.01 vs. the C group; ++ p<0.01 vs. (-)-OSU6162. Fig 8 shows that, similar to drug naive mice, (-)-and (+)-OSU6162 decreased motor activity in active rats. Again, the (-)-form was more potent than the (+)-form and the (-)-form was also more efficient than the (+)-form in inhibiting motor activity.

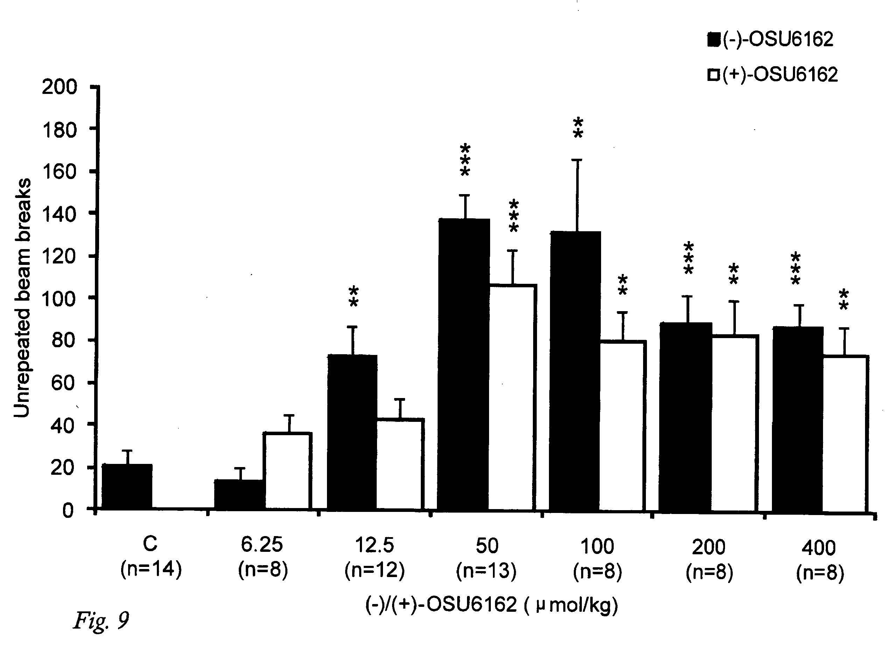

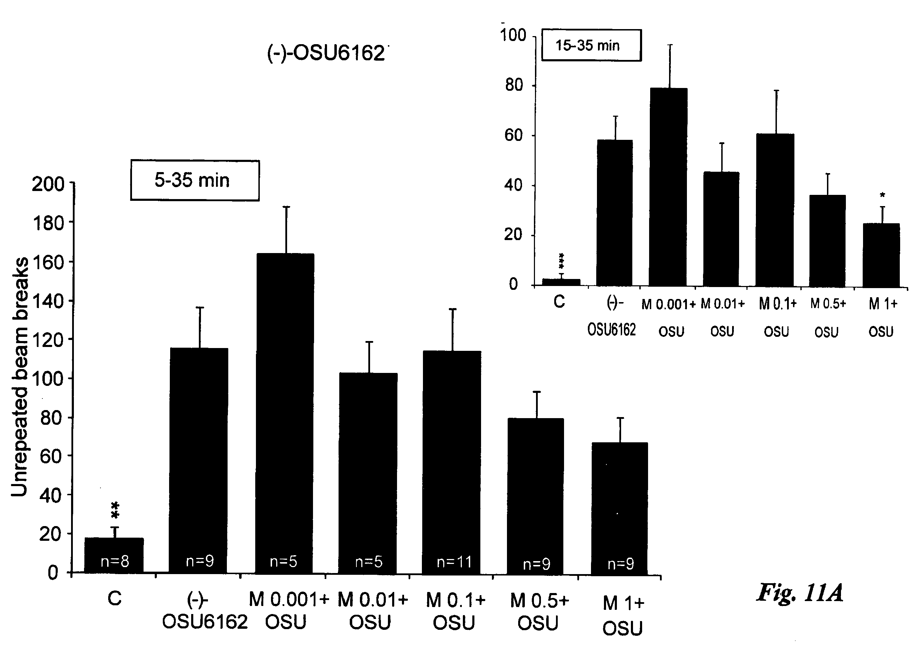

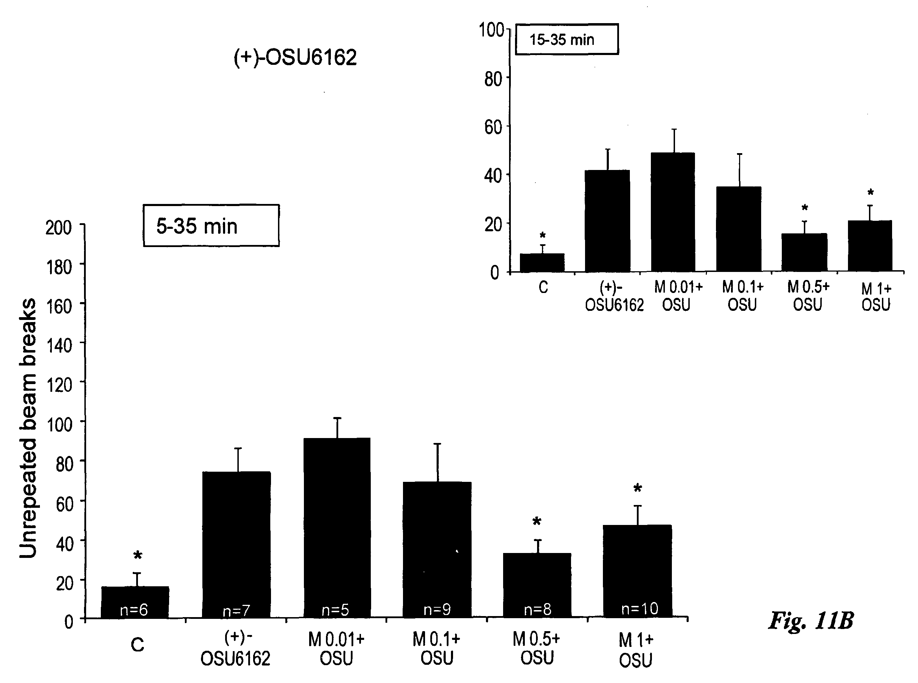

Conversely, in habituated rats with a low activity level, both (-)- and (+)-OSU6162 stimulated motor activity (Fig 9). Locomotion was measured as accumulated unrepeated beam breaks. Rats were allowed to habituate for 65 minutes and then injected with (-)-OSU6162, (+)-OSU6162 (sc) or NaCl (Control = C, 0.9%, sc). This was followed by 60 minutes of registration. Data are calculated from a 30 minutes period, 5-35 minutes after injection, and plotted as means and SEM. Statistical comparisons were made by Mann-Whitney U-test vs. the Control group, ** p<0.01, *** p<0.001. The OSU6162 enantiomers were administered in the doses 6.25, 12.5, 50, 100, 200 and 400 µmol/kg. For (-)-OSU6162 significant locomotor stimulation was observed for all doses but the lowest; for (+)-OSU6162 significant locomotor stimulation was observed for all doses but the two lowest. When animals (controls and rats treated with 50 µmol/kg of (-)-OSU6162 and (+)-OSU6162, respectively) from experiments shown in figures 9 and 11 were pooled, Mann-Whitney U-test showed that (-)-OSU6162 caused a greater degree of locomotor stimulation than (+)-OSU6162. Mean ± SEM and number of animals (n) per pooled group were for controls 23±6 (n=25), for (-)-OSU6162 129±11 (n=22) and for (+)-OSU6162 95±12 (n=20). (-)-OSU6162 and (+)-OSU6162 vs controls: p < 0.001; (-)-OSU6162 vs (+)-OSU6162: p < 0.05. Data were calculated from a 30 minutes period, 5-35 minutes after injection.

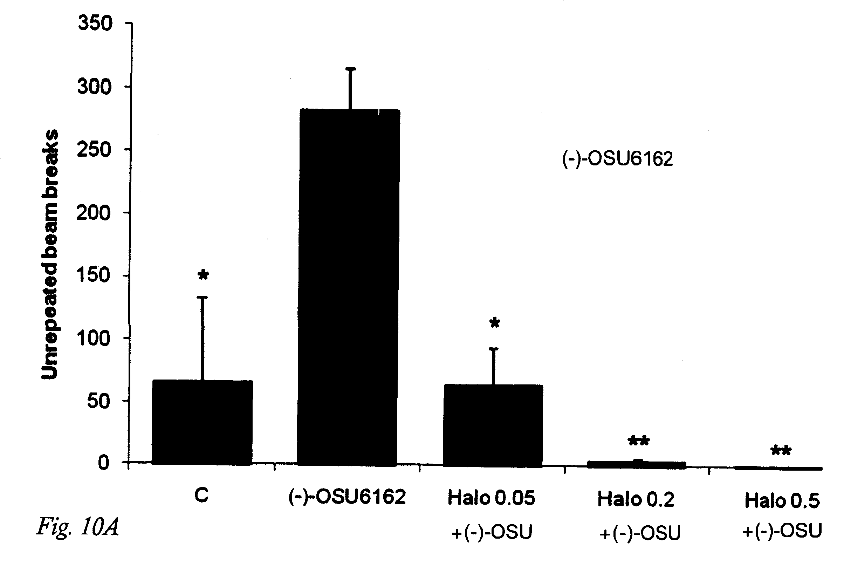

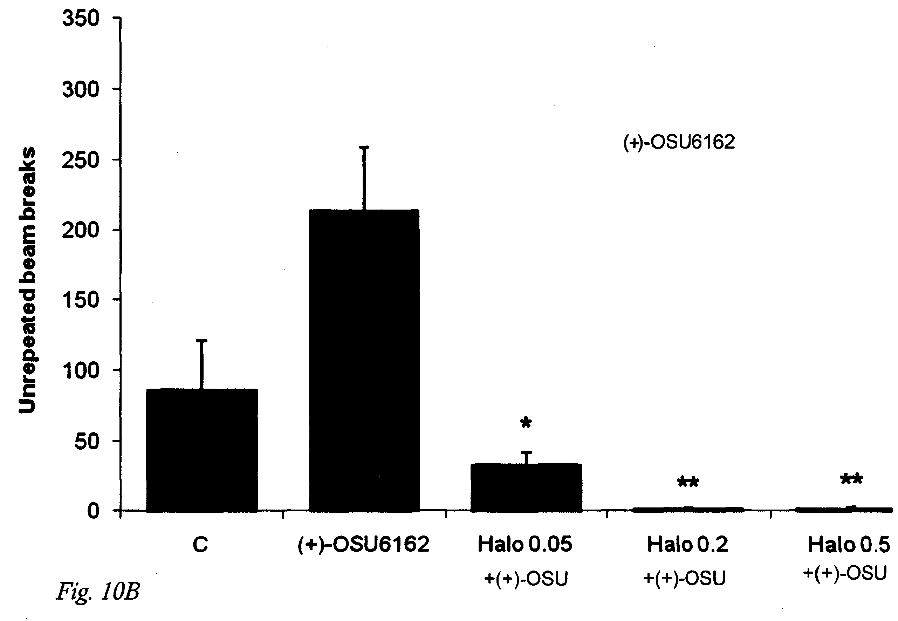

The effect of haloperidol on the locomotor stimulation was also studied in habituated rats (see figure 10). Locomotion was measured as accumulated unrepeated beam breaks. Rats were given haloperidol (0.05, 0.2, 0.5 mg/kg, sc) and were then allowed to habituate for 65 minutes. After habituation rats were injected with (-)-OSU6162 or (+)-OSU6162 (both 50 µmol/kg, sc) and then recorded for 60 minutes. Data are calculated from a 30 minutes period, taken 5-35 minutes after injection, and plotted as means and SEM. N = 4-5. Statistical comparisons were made by Mann-Whitney U-test vs. a) (-)-OSU6162 group and b) (+)-OSU6162 group, * p<0.05, ** p<0.01. Fig 10 shows that haloperidol (0.05, 0.2 and 0.5 mg/kg) effectively antagonized the locomotor stimulation induced by (-)- and (+)-OSU6162 (both 50 µmol/kg, sc) in habituated rats.

The effect of M100907 on the locomotor stimulation induced by a) (-)-OSU6162 and b) (+)-OSU6162 in habituated rats was also studied (See Figure 11). Locomotion was measured as accumulated unrepeated beam breaks. Rats were given M100907 (ip) and were then allowed to habituate for 65 minutes. After habituation rats were injected with (-)-OSU6162 or (+)-OSU6162 (both 50 µmol/kg, sc) and then recorded for 60 minutes. Data are calculated from a 30 minutes period, taken 5-35 minutes (inserted figures: 15-35 minutes) after injection, and plotted as means and SEM. Statistical comparisons were made by Mann-Whitney U-test vs. a) (-)-OSU6162 group and b) (+)-OSU6162 group, * p<0.05, ** p<0.01. Fig 11 shows that in the 5-35 minute interval 0.5 and 1 mg/kg of M100907 significantly counteracted the locomotor stimulation induced by (+)- but not by (-)-OSU6162 (both 50 µmol/kg, sc) in habituated rats. In the 15-35 minute interval, however there was a significant reversal of the (-)-OSU6162-induced locomotor stimulation, too, following administration of 1 mg/kg of M100907.

### In vitro experiments

### Materials and methods

NIH-3T3 cells (CRL 1658) and human embryonic kidney 293T (HEK-293T, CRL 11268) cells were purchased from American Tissue Culture Collection. O-nitrophenyl-beta-D-galactopyranoside and nonidet P-40 were from Sigma. Tissue culture media used was Dulbecco's modified Eagles medium (DMEM) (Gibco-BRL) 96-well tissue culture dishes were from Falcon. Hanks balanced salt solution without magnesium chloride, magnesium sulfate, and calcium chloride, Trypsin-EDTA were all from Gibco-BRL.

### Drugs

All compounds for in vitro studies were solubilized as 10 mM stock solutions in either water or DMSO. Working dilutions were made from 50 µM solutions in DMEM with 25% Ultraculture, 1% PSG. 3-PPP is [3-(3-hydroxyphenyl)-N-n-propyl]piperidine. 5-CT is 5-carboxamidotryptamine. 5-HT is 5-hydroxytryptamine. All compounds were obtained from Sigma/RBI (Natick, MA) except as follows: N-desmethylclozapine (8-chloro-11-(1-piperazinyl)-5H-dibenzo [b,e] [1,4] diazepine) also called NDMC and aripiprazole (7-{4-[4-(2,3-Dichloro-phenyl)-piperazin-1-yl]-butoxy}-3,4-dihydro-1H-quinolin-2-one) were synthesized atACADIA. Pramipexol ((6S)-N⁶-propyl-4,5,6,7-tetrahydro-1,3-benzothiazole-2,6-diamine) was obtained as prescription tablets.

### Cell culture

NIH-3T3 cells were incubated at 37 °C in a humidified atmosphere (5% CO2) in DMEM supplemented with 4500-mg/l glucose, 4 nM L-glutamine, 50 U/ml penicillin G, 50 U/ml streptomycin (PSG, HyClone from Fisher Scientific Logan, UT) and 10% calf serum. HEK-293T cells were incubated at 37°C in a humidified atmosphere (5% CO₂) in Dulbecco's modified Eagles tissue culture medium with the same supplements used for NIH 3T3 cells except plus 10% Fetal calf serum was used instead of calf serum.

### Constructs

The human D1, D2 (short form), D3, D4 (variant 4.2), D5, 5-HT1A, 5-HT1 B, 5-HT1D, 5-HT1 E, 5-HT1 F, 5-HT2A, 5-HT2B, 5-HT2C(vgv), 5-HT6, 5-HT7, alpha1A, alpha1B, alpha2A, alpha2B, alpha2C, M1, M2, M3, M4, M5 and H1 receptors have been described previously Burstein ES, Ma J, Wong S, Gao Y, Pham E, Knapp AE, Nash NR, Olsson R, Davis RE, Hacksell U, Weiner DM, Brann MR. (2005) Intrinsic efficacy of antipsychotics at human D2, D3, and D4 dopamine receptors: identification of the clozapine metabolite N-desmethylclozapine as a D2/D3 partial agonist. J Pharmacol Exp Ther. 315:1278-1287; Spalding TA, Trotter C, Skjaerbaek N, Messier TL, Currier EA, Burstein ES, Li D, Hacksell U and Brann MR. (2002) Discovery of an ectopic activation site on the M(1) muscarinic receptor. Mol Pharmacol 61:1297-1302; Weiner DM, Burstein ES, Nash N, Croston GE, Currier EA, Vanover KE, Harvey SC, Donohue E, Hansen HC, Andersson CM, Spalding TA, Gibson DF, Krebs-Thomson K, Powell SB, Geyer MA, Hacksell U, Brann MR. (2001) 5-hydroxytryptamine2A receptor inverse agonists as antipsychotics. J Pharmacol Exp Ther. 299:268-276). The human dopamine transporter (DAT), serotonin transporter (SERT), and norepinephrine transporter (NET) were cloned by PCR. All clones were subcloned into the pSI vector (Promega Corp., Madison, WI) and sequence verified before use.

### Receptor Selection and Amplification Technology (R-SAT^{™}) assay

R-SAT^{™} assays were performed as described (Burstein et al, 2005 (as above); Burstein ES, Piu F, Ma JN, Weissman JT, Currier EA, Nash NR, Weiner DM, Spalding TA, Schiffer HH, Del Tredici AL, Brann MR. (2006) Integrative functional assays, chemical genomics and high throughput screening: harnessing signal transduction pathways to a common HTS readout. Curr Pharm Des. 12:1717-1729; Ma JN, Owens M, Gustafsson M, Jensen J, Tabatabaei A, Schmelzer K, Olsson R, Burstein ES. (2011) Characterization of Highly Efficacious Allosteric Agonists of the Human Calcium-Sensing Receptor. J Pharmacol Exp Ther.) with the following modifications. The data for 5-HT2A, D2, D3 and D4 receptors were generated as follows: Cells were plated one day before transfection using 7x10³ cells in 0.1 ml of media per well of a 96-well plate. Cells were transiently transfected with 1 to 10 ng/well of receptor DNA, and 30 ng/well pSI-beta-galactosidase (Promega, Madison, WI) per well of a 96-well plate using Polyfect (Qiagen, Valencia, CA) according to the manufacturer's instructions. For D2, D3, and D4 assays, 20 ng/well ras/rap1 B(AA), 2 ng/well adenylyl cyclase 2; and 5 ng/well each of the G-proteins Gαo, Gß1, and Gγ2 were additionally transfected as described (Burstein et al, 2005 (as above)). One day after transfection media was changed and cells were combined with ligands in DMEM supplemented with 25% Ultraculture synthetic supplement (Cambrex, Walkersville, MD) instead of calf serum to a final volume of 200 µl/well. After five days in culture beta-galactosidase activity was measured and responses quantified on a plate-reader (Bio-Tek EL 310 or Molecular Devices). All data were analyzed using the computer programs Excel Fit and GraphPad Prism software (San Diego). The data for 5-HT1A, 5-HT1 B, 5-HT1D, 5-HT1 E, 5-HT1 F, 5-HT2B, 5-HT2C(vgv), 5-HT6, 5-HT7, alpha1A, alpha1B, alpha2A, alpha2B, alpha2C, M1, M2, M3, M4, M5 and H1 receptors was generated using a similar method. Briefly, NIH/3T3 cells grown in larger volumes (632 cm2 cell factory flasks, Nalgene Nunc International, Rochester, NY) to 70% confluency were transfected with DNA encoding beta-galactosidase, the individual human receptors as described in the text, and "helper" DNAs encoding accessory proteins such as chimeric G-proteins to enable responses to Gi-coupled and Gs-coupled receptors (Spalding et al, 2002 (as above); Weissman JT, Ma JN, Essex A, Gao Y and Burstein ES. (2004) G-protein-coupled receptor-mediated activation of rap GTPases: characterization of a novel Galphai regulated pathway. Oncogene 23: 241-249.; Burstein et al, 2006 (as above)) using Polyfect (Qiagen, Valencia, CA) as per manufacturer's instructions. Transfected cells were frozen at -80°C in DMEM containing 10% calf serum and 10% dimethyl sulfoxide using 5100 Cryo Freezing containers (Nalgene Labware, Rochester, NY), and subsequently transferred to -135°C for long-term storage. On day of the assay, cells were thawed and added in DMEM containing 30% Ultraculture (Lonza, Basel, Switzerland) and 0.4% calf serum (Hyclone, Logan, UT) directly to ligands at varying concentrations on 96-well tissue culture plates. After five days in culture, plates were processed as described above.

### Bioluminescence Resonance Energy Transfer (BRET-2) Assays

BRET-2 assays were performed as described (Ma JN, Schiffer HH, Knapp AE, Wang J, Wong KK, Currier EA, Owens M, Nash NR, Gardell LR, Brann MR, Olsson R, Burstein ES. (2007) Identification of the atypical L-type Ca2+ channel blocker diltiazem and its metabolites as ghrelin receptor agonists. Mol Pharmacol. 72:380-386) except that a mutant form of beta-arrestin 2 truncated at L373 was used for D2 assays, using HEK-293T cells transfected with the Fugene HD transfection kit (Roche, Palo Alto, USA) according to the manufacturer's instructions. BRET-2 signals were measured using the multiplate reader Mithras 940LB (Berthold Technologies, Bad Wildbad, Germany) and were calculated as the ratio between the *Renilla luciferase* emission and the GFP2 emission corrected by the background emissions of non-transfected cells.

### Membrane preparations

Membranes were made as previously reported (Ma et al, 2007 (as above)) with the following modifications: HEK-293T cells were seeded at 13.5x10⁶ cells per 15cm dish and were transfected 24 hrs later by mixing 11 µg of DNA in 900 µl DMEM, adding 33 µl FuGENE (Roche Applied Science, Indianapolis, IN) dropwise, incubating the mixture for 15 minutes at room temperature, and adding it to the plate. Cells were not centrifuged following cell scraping, but were collected directly into the ice-cold nitrogen cavitation chamber.

### GTPγS-binding assays

GTPγS binding assays were performed as described previously (Burstein ES, Ott TR, Feddock M, Ma JN, Fuhs S, Wong S, Schiffer HH, Brann MR, Nash NR. (2006b) Characterization of the Mas-related gene family: structural and functional conservation of human and rhesus MrgX receptors. Br J Pharmacol. 147:73-82) with the following modifications: 5 µg of membranes were incubated at 24°C on an orbital shaker at 100 RPM for 1 hour in assay buffer (20 mM HEPES, 100 mM NaCl, 5 mM MgCl₂, pH 7.4) in the presence of 10 µM GDP, 0.4 nM ³⁵S-GTP-γS (Perkin Elmer Life Sciences, Shelton, CT) and varying concentrations of agonist (total volume 200 µl in a 96 well plate Pico Plate (Perkin Elmer, Shlton, CT)). 50 µl/well Wheat-Germ Agglutinin SPA beads (Perkin Elmer, Shelton, CT) were added to the plates and the plates incubated for 1 hour. The plates were then centrifuged at 1,000 x g for 10 minutes and read on a Top Count NXT (Perkin Elmer, Shelton, CT).

### Phosphatidyl Inositol Hydrolysis assays

PI hydrolysis assays were performed as described previously (Gardell LR, Ma JN, Seitzberg JG, Knapp AE, Schiffer HH, Tabatabaei A, Davis CN, Owens M, Clemons B, Wong KK, Lund B, Nash NR, Gao Y, Lameh J, Schmelzer K, Olsson R, Burstein ES. (2008) Identification and characterization of novel small-molecule protease-activated receptor 2 agonists. J Pharmacol Exp Ther. 327:799-808). Briefly, HEK-293T cells were seeded at 4.2 million in 10cm dish and transfected next day by mixing 10µg of DNA in 500µl DMEM and 30µl FuGene HD Transfection Reagent (Roche). After lysing the cells with 50µl of 0.1 M formic acid, 20µl each cell lysate was mixed with 80µl (1mg) RNA Binding YSi-SPA beads (GE Healthcare, Chalfont St. Giles, UK) on PICO plates (PerkinElmer) and counted on a TopCount (Packard).

### Equilibrium radioligand binding to monoamine transporters

Membranes were prepared as described above. For binding assays, membranes expressing DAT (0.8 µg/well), SERT (5 µg/well), and NET (5 µg/well) were incubated with 50 pM ¹²⁵I-RTI-55 (for DAT) or 1 nM ³H-Imipramine (NET and SERT) for 2 h at room temperature in binding buffer (SERT: 50 mM Tris, 120 mM NaCl, 5 mM KCI, pH 7.4; NET: 50 mM Tris, 300 mM NaCl, 5 mM KCl, pH 7.4; DAT: 50mM Tris, 120mM NaCl, pH7.4). Binding reactions were terminated by filtration through UniFilter-96GF/B filters (from Perkin-Elmer, presoaked with 0.1 % polyethylenimine for 1 h) with a Brandel 96-well harvester. Filters were washed with ice-cold wash buffer (50 mM Tris, 120mM NaCl, pH7.4; 500 ml/plate) and then allowed to air-dry for 30min. 50 µl MacrosScint-20 scintillation cocktail was added to each well, the plates were sealed, and then counted on a Top-Count (Packard).

### Data analysis

Agonist curves from R-SAT^{™} , GTP□S, and BRET experiments were fitted to a sigmoidal dose-response function: Y=B+(T-B)/(1+10^(LogEC50-LogX))) where Y is the response, B is the baseline, T is the top or maximum response, and X is the concentration of ligand. The Schild plot was obtained by plotting log(DR-1) versus log antagonist ligand concentration and fitting the data to a straight line function using linear regression. DR represents the dose ratio of the EC₅₀ of dopamine in the presence of the indicated concentration of (-)-OSU6162 over the EC₅₀ of dopamine alone. Data for off-rate assays were fitted to a 1-phase mono-exponential decay equation: Y=(T-B)exp(-kX) + B where Y is the amount of bound ligand remaining at time = X starting at T at time = 0 and ending at B. All data analysis was performed using GraphPad Prism version 4.0 (San Diego, CA).

### Results

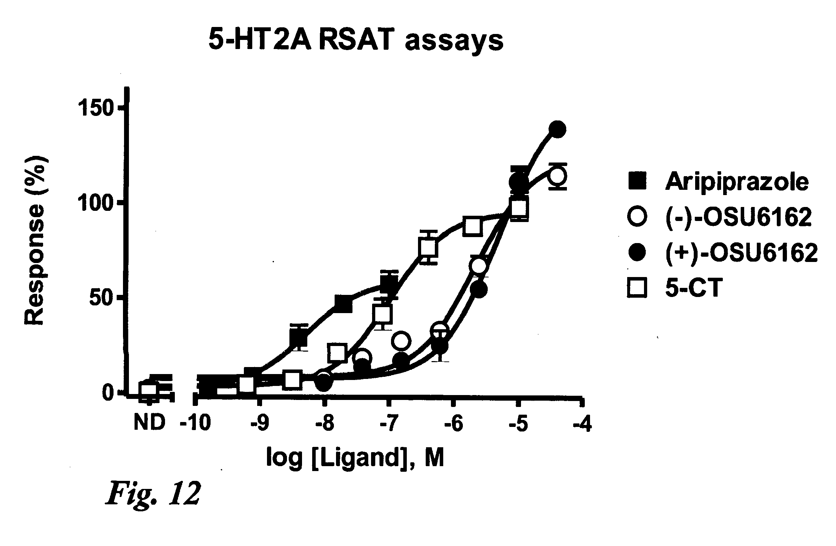

The results from the behavioral experiments in rats and mice described above prompted a series of functional assays to profile both enantiomers of OSU6162 at 5-HT2A receptors. (Receptor Selection and Amplification Technology (R-SAT^{™}) assays were carried out using human 5-HT2A receptors transiently expressed in NIH3T3 cells as described in the methods using the indicated concentrations of aripiprazole (filled squares), 5-CT (open squares), (-)-OSU-6162 (filled circles) and (+)-OSU6162 (open circles). Each data point is the mean of two determinations from one independent experiment. Each curve is representative of at least three or more independent experiments. Responses were normalized to the response to 5-CT which was assigned a value of 100%. Responses to 5-CT were typically 10 fold over baseline. Figure 12 shows that in the R-SAT^{™} cellular proliferation assay (Burstein et al, 2005, 2006 (both as above); Ma et al 2011 (as above)), both enantiomers of OSU6162 were full agonists at 5-HT2A receptors, with the (+)-enantiomer showing slightly greater efficacy than the (-)-enantiomer (see also Table 1).

**Table 1. Functional profile at 5-HT2A receptors**

| | **R-SAT^{™}** | | **PI Hydrolysis** | | **BRET2** | |
|---|---|---|---|---|---|---|
| **Ligands** | **pEC50** | **Eff (%)** | **pEC50** | **Eff (%)** | **pEC50** | **Eff%** |
| **5-HT** | **7.3 ± 0.2** | **93 ± 20** | **8.0 ± 0.3** | **100 ± 0** | **8.1 ± 0.1** | **100 ± 5** |
| **5-CT** | **7.2 ± 0.3** | **100 ± 0** | **6.6 ± 0.0** | **92 ± 4** | **6.7 ± 0.1** | **98 ± 3** |
| **Pergolide** | **8.9 ± 0.9** | **104 ± 28** | **9.1 ± 0.0** | **96 ± 8** | **7.6 ± 0.4** | **124 ± 4** |
| **Quinpirole** | **5.9 ± 0.3** | **131 ± 14** | **5.6 ± 0.1** | **88 ± 13** | **5.4 ± 0.1** | **91 ± 5** |
| **(+)-OSU-6162** | **5.8 ± 0.3** | **139 ± 14** | **5.7 ± 0.1** | **82 ± 4** | **4.8 ± 0.1** | **84 ± 2** |
| **(-)-OSU-6162** | **6.2 ± 0.4** | **113 ± 18** | **5.9 ± 0.2** | **65 ± 4** | **5.0 ± 0.1** | **63 ± 2** |
| **Pramipexole** | **5.9 ± 0.5** | **48 ± 9** | **nd** | **nd** | **4.7 ± 0.1** | **52 ± 3** |
| **(+)-3-PPP** | **5.2 ± 0.4** | **87 ± 26** | **<5.0 -** | **17 -** | **<4.0 -** | **42 ± 5** |
| **(+)-Terguride** | **9.8 ± 0.9** | **89 ± 13** | **9.1 ± 0.2** | **52 ± 8** | **7.0 ± 0.0** | **42 ± 2** |
| **Aripiprazole** | **8.2 ± 0.3** | **68 ± 14** | **7.9 ± 0.1** | **26 ± 0** | **6.6 ± 0.3** | **30 ± 7** |
| **(-)-3-PPP** | **5.4 ± 0.5** | **47 ± 12** | - | **5 -** | **-** | **10 ± 4** |

Values are the mean ± SEM of at least three (Receptor Selection and Amplification Technology, R-SAT^{™}), or two (Phosphatidyl Inositol (PI) hydrolysis or Bioluminescence Resonance Energy Transfer (BRET2))) or more independent experiments. A dash (-) indicates not calculated. nd indicates not done. Potency is reported as the negative logarithm of EC₅₀ in molar (M). Efficacy is reported as percent response of the reference ligand which was 5-CT (R-SAT^{™}) or 5-HT (PI hydrolysis and BRET2). 100% represents 6-10 fold responses in RSAT, 5-fold responses in PI hydrolysis assays, and 2-fold responses in BRET2 assays.

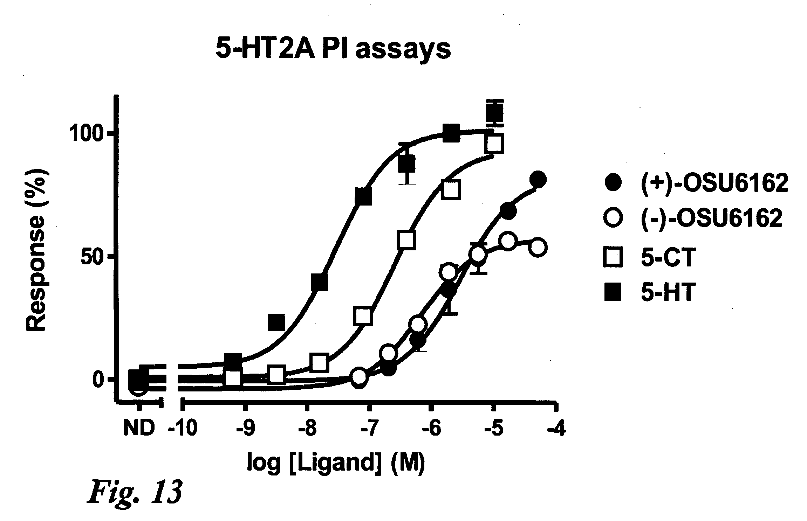

To relate the results observed in R-SAT^{™} to a more conventional assay system, the same ligands were tested in phosphatidyl inositol (PI) hydrolysis assays (Figure 13). PI assays were carried out using human 5-HT2A receptors transiently expressed in HEK 293T cells as described in the methods using the indicated concentrations of (-) OSU-6162 (open circles), (+) OSU-6162 (filled circles), 5-CT (open squares) and 5-HT (filled squares). Each data point is the mean of two determinations from one independent experiment. Each curve is representative of at least three or more independent experiments. Responses were normalized to the response to 5-HT which was assigned a value of 100%. Responses to 5-HT were typically 6 to 10 fold over baseline. Very similar results were observed, however in the PI assays both compounds were partial agonists (Figure 13, Table 1). (+)-OSU6162 again showed higher efficacy than the (-) enantiomer, but the (-) enantiomer was slightly more potent.

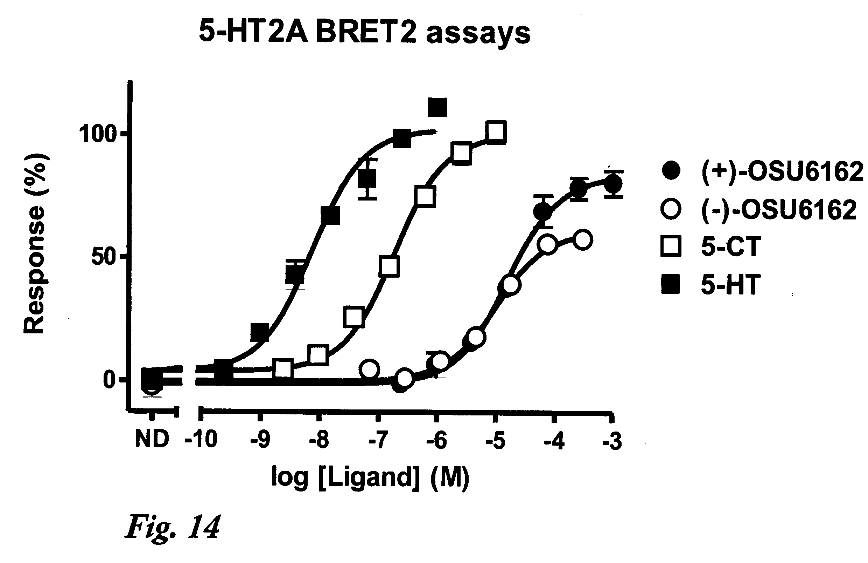

Bioluminescence resonance energy transfer (BRET2) assays (Figure 14) were carried out using human 5-HT2A receptors carboxy terminally tagged with *Renilla* luciferase and transiently co-expressed with GFP2 tagged beta-arrestin-2 in HEK 293T cells as described in the methods using the indicated concentrations of (-) OSU-6162 (open circles), (+) OSU-6162 (filled circles), 5-CT (open squares) and 5-HT (filled squares).

Each data point is the mean of two determinations from one independent experiment. Each curve is representative of at least two or more independent experiments. Responses were normalized to the response to 5-HT which was assigned a value of 100%. Responses to 5-HT were typically 2 fold over baseline. Both enantiomers effectively induced beta-arrestin-2 recruitment to 5-HT2A receptors, with maximum responses of approximately 60 and 80 percent of 5-HT for the (-) and (+) enantiomers, respectively (Figure 14, Table 1).

Because some antipsychotic drugs and anti-Parkinsonian drugs are reported to have agonist actions at 5-HT2A receptors, and since it was observed that both enantiomers of OSU6162 do too, it was decided to benchmark the 5-HT2A activity of (-)-OSU6162 more precisely by profiling it along with many other antipsychotic and anti-Parkinsonian drugs in R-SAT^{™}, phosphatidyl inositol (PI) hydrolysis, and BRET2 assays. As shown in Table 1, most of the agents tested did show appreciable activity at 5-HT2A receptors in R-SAT, and to lesser degrees in PI and BRET2 assays. However their potencies at 5-HT2A receptors were typically 100 to 1000 fold lower than at D2 receptors (see Table 2 for D2). In contrast, (-)-OSU6162 was equipotent at D2 and 5-HT2A receptors, and (+)-OSU6162 was more potent at 5-HT2A than D2 receptors.

**Table 2. Functional profile at D2 receptors.**

| | **R-SAT^{™}** | | **GTPγS** | | **BRET2** | |
|---|---|---|---|---|---|---|
| Ligand | pEC50 | Eff(%) | pEC50 | Eff% | pEC50 | Eff% |
| Dopamine | nd | nd | 8.0 ± 0.1 | 100 ± 0 | 6.3 ± 0.1 | 100 ± 0 |
| Pramipexole | 9.3 ± 0.4 | 84 ± 9 | 8.4 ± 0.2 | 87 ± 6 | 6.1 ± 0.1 | 104 ± 1 |
| Quinpirole | 9.0 ± 0.1 | 98 ± 6 | 8.1 ± 0.2 | 92 ± 5 | 6.2 ± 0.0 | 69 ± 1 |
| Pergolide | 9.8 ± 0.1 | 100 ± 0 | 9.0 ± 0.1 | 94 ± 7 | 6.7 ± 0.2 | 64 ± 3 |
| (+)-3-PPP | 7.4 ± 0.1 | 101 ± 8 | 7.1 ± 0.0 | 102 ± 4 | 5.3 ± 0.1 | 58 ± 2 |
| (+)-Terguride | 11.1 ± 0.2 | 92 ± 20 | 9.3 ± 0.2 | 88 ± 8 | 7.2 ± 0.1 | 8 ± 1 |
| (-)-3-PPP | 8.0 ± 0.1 | 77 ± 4 | 7.7 ± 0.1 | 77 ± 4 | 6.5 ± 0.2 | 2 ± 0 |
| Aripiprazole | 10.5 ± 0.1 | 62 ± 3 | 8.1 ± 0.2 | 34 ± 6 | - | 0 ± 0 |
| (-)-OSU-6162 | 6.1 ± 0.3 | 36 ± 3 | 6.3 ± 0.1 | 37 ± 9 | - | 0 ± 0 |
| (+)-OSU-6162 | - | *77 ± 5 | - | *36 ± 0 | - | 0 ± 0 |
| NDMC | 7.9 + 0.1 | 33+2 | 7.4 ± 0.2 | 18 ± 1 | - | 0 ± 0 |

Values are the mean ± SEM of at least five (Receptor Selection and Amplification Technology, R-SAT^{™}), three (GTPγS), or two (Bioluminescence Resonance Energy Transfer (BRET2)) or more independent experiments. A dash (-) indicates not calculated. nd indicates not done. Potency is reported as the negative logarithm of EC₅₀ in molar. Efficacy is reported as percent response of the reference ligand which was pergolide (RSAT) or dopamine (GTPγS and BRET2). 100% represents 6-10 fold responses in RSAT, 3-fold responses in GTPγS binding assays, and 3-fold responses in BRET2 assays. *response at 50 µM, the maximum concentration tested.

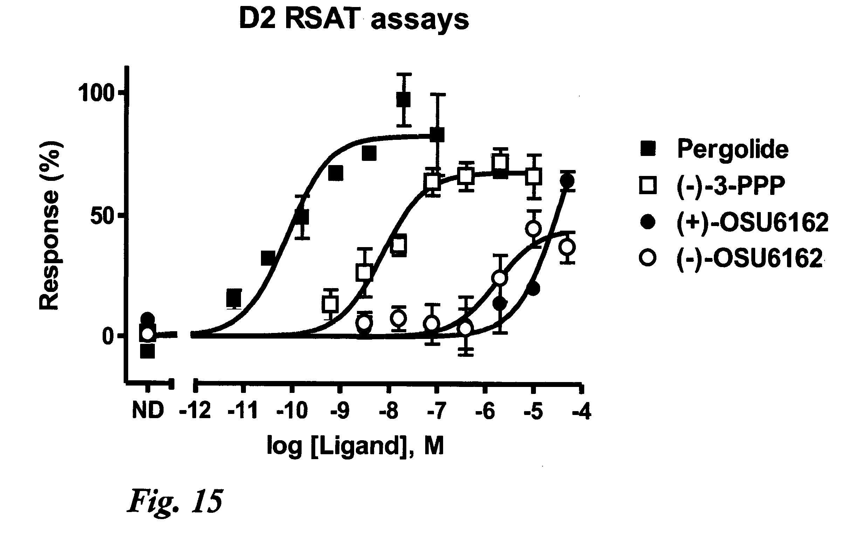

(-)-OSU6162 has been reported to be a D2 partial agonist, therefore both enantiomers of OSU6162 at D2 receptors were also profiled. As described previously (Burstein et al, 2005, 2006 (as above)), the R-SAT^{™} functional assay is able to detect functional responses even from partial agonists with very low intrinsic activity. Therefore, (-)-OSU6162 along with a collection of dopaminergic ligands for agonist activity at the human D2 dopamine receptor in R-SAT^{™} were also tested. Because dopamine is relatively unstable in the R-SAT^{™} assay, pergolide was used as the reference ligand for these studies. In Figure 15A) R-SAT^{™} assays were carried out using human D2 receptors transiently expressed in NIH3T3 cells as described in the methods using the indicated concentrations of pergolide (filled squares), (-)-3-PPP (open squares), (+)-OSU-6162 (filled triangles) and (-)-OSU6162 (open circles). Each data point is the mean of two determinations from one independent experiment. Each curve is representative of at least five or more independent experiments. Responses were normalized to the response to pergolide which was assigned a value of 100%. Responses to pergolide were typically 6 fold over baseline. Compared to pergolide, (-)-OSU6162 had 30% efficacy, very similar to N-desmethylclozapine (NDMC) (Figure 15, Table 2). The (+)-enantiomer of OSU6162 was less potent but appeared to have higher efficacy than the (-)-enantiomer, however it was not possible to determine its maximum response at the concentrations tested. Other ligands previously characterized as D2 partial agonists such as aripiprazole and (-)-3-PPP had partial agonist activity in R-SAT^{™} with 62% and 77% efficacy, respectively. All other ligands tested had full or nearly full efficacy in R-SAT^{™} (Table 2).

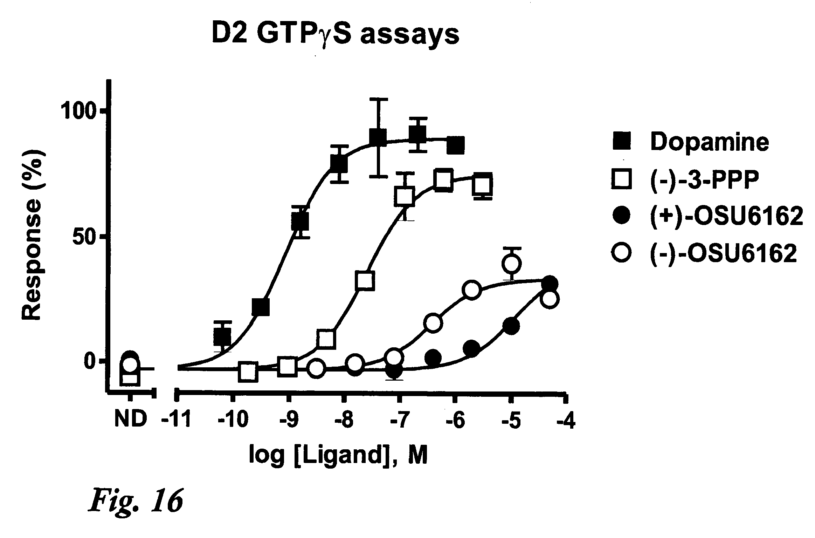

To relate the results observed in R-SAT^{™} to a more conventional assay system, these ligands were also tested for the ability to induce GTPγS binding through D2 receptors. This assay format also allowed a more direct comparison with dopamine itself (Figure 16). GTPγS assays were carried out using human D2 receptors transiently expressed in HEK293T cells as described in the methods using the indicated concentrations of dopamine (black squares), (-)-3-PPP (open squares), (-)-OSU-6162 (open circles) and (+)-OSU-6162 (filled circles). Each data point is the mean of two determinations from one independent experiment. Each curve is representative of at least two or more independent experiments. Responses were normalized to the response to dopamine which was assigned a value of 100%. Responses to dopamine were typically 3 fold over baseline.

As seen in R-SAT^{™}, the (-)-enantiomer was significantly more potent than the (+)-enantiomer, both were partial agonists, and again the (+)-enantiomer did not reach its maximum response at the concentrations tested suggesting it may have higher efficacy (Figure 16). Overall the rank order of activity observed in GTPγS binding assays was very similar to that observed in R-SAT^{™} assays with slightly lower efficacies observed for some compounds, especially NDMC and aripiprazole (Table 2). (-)-3-PPP and (+)-terguride remained strong partial agonists, while (+)-3-PPP remained a full agonist.

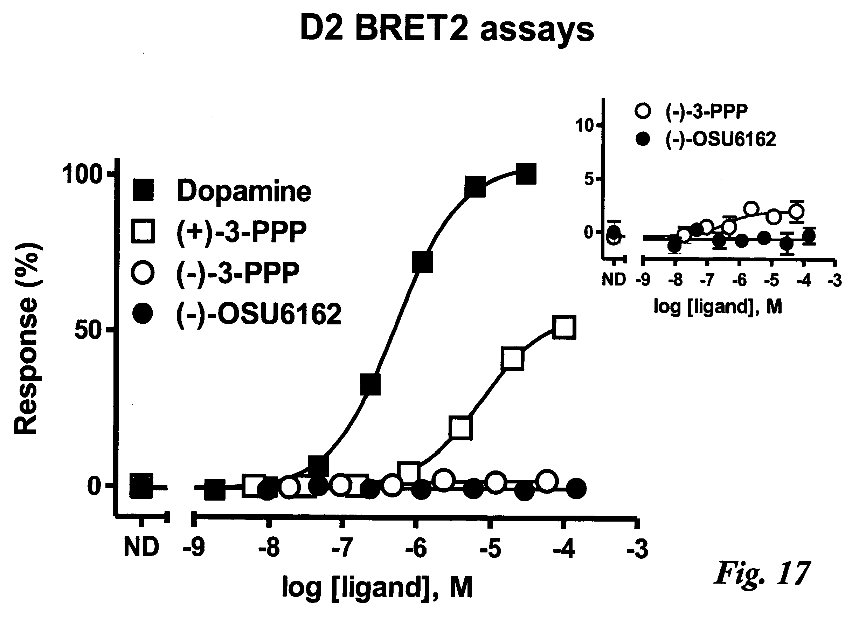

Bioluminescence resonance energy transfer (BRET2) assays were constructed using luciferase-tagged human D2 receptors and green fluorescent protein (GFP) - tagged beta arrestin 2 to monitor agonist-induced beta-arrestin recruitment by D2 receptors (Figure 17). BRET2 assays were carried out using human D2 receptors carboxy terminally tagged with *Renilla* luciferase and transiently co-expressed with GFP2 tagged β-arrestin-2 in HEK293T cells as described in the methods using the indicated concentrations of dopamine (filled squares), (+)-3-PPP (open squares), (-)-3-PPP (open circles), and (-)-OSU-6162 (filled circles). Each data point is the mean of two determinations from one independent experiment. Each curve is representative of two to three independent experiments. Responses were normalized to the response to dopamine which was assigned a value of 100%. Responses to dopamine were typically 3 fold over baseline.

Since this assay configuration monitors protein-protein interactions, and since there is no opportunity for signal transduction amplification as in R-SAT^{™} and GTPγS assays, one can evaluate the intrinsic activities of agonists in an assay system with little receptor reserve. A much sharper discrimination of intrinsic activity was observed in this assay system (Figure 17, Table 2). In BRET2, compounds with partial agonist profiles in the R-SAT^{™} and GTPγS assays, including both enantiomers of OSU6162, NDMC and aripiprazole, had no agonist activity in BRET2 assays. (-)-3-PPP and (+)-terguride, which were nearly full agonists in R-SAT^{™}, were reduced to responses of 2% and 8%, respectively in BRET2, whereas ligands with full efficacy in R-SAT^{™} and GTP□S assays such as pergolide, (+)-3-PPP, and quinpirole, were partial agonists in BRET2 assays. Only pramipexole remained a full agonist in BRET2 assays.

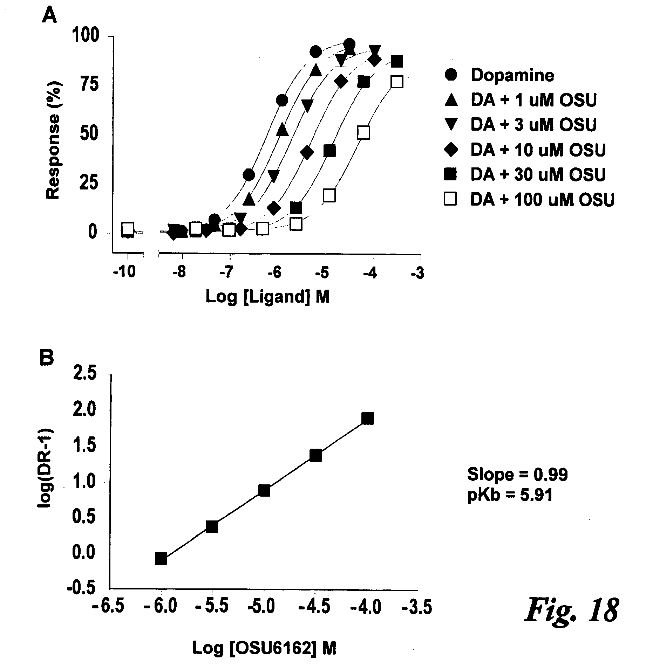

The region of the receptor to which the endogenous agonist binds is termed the orthosteric site whereas other ligands may act through allosteric, or non-overlapping sites. (-)-OSU6162 has been described as a 'dopamine stabilizer', interacting both orthosterically and allosterically with the D2 receptor; more recently others have found some, albeit not strong, support for allosteric effects of (-)-OSU6162 on the D2 receptor. To examine whether or not (-)-OSU6162 acts allosterically at D2 receptors, increasing concentrations of the compound as a functional antagonist of dopamine in BRET2 assays (Figure 18) were tested. BRET2 assays were carried out as described above using serial dilutions of dopamine together with the indicated concentrations of (-)-OSU6162. A Schild plot (B) was constructed using the EC₅₀ values derived from the curves in (A). The Schild plot was fitted using linear regression in GraphPad Prizm.

The Schild analysis yielded a slope of nearly unity, indicating a competitive interaction of (-)-OSU6162 with dopamine at D2 receptors, suggesting (-)-OSU6162 binds orthosterically to D2 receptors. The pKb of (-)-OSU6162 was estimated to be 5.9 from the Schild analysis, which is consistent with the pEC₅₀ estimates of 6.1 and 6.3 in the R-SAT^{™} and GTPγS assays, respectively. Similarly, it was observed that (-)-OSU6162 did not significantly alter the dissociation rate of dopamine from D2 receptors (data not shown), also suggesting (-)-OSU6162 does not allosterically modulate D2 dopamine receptors.

To gain further insights into the mechanistic basis for the actions of (+)-OSU6162 and (-)-OSU6162, these compounds were profiled at a variety of other monoaminergic receptors and transporters (Table 3).

**Table 3. Profile of (-)-OSU6162 and (+)-OSU6162.**

| | **(-)-OSU6162** | | **(+)-OSU6162** | | | **Reference** | | |
|---|---|---|---|---|---|---|---|---|
| **Receptor** | **pEC50** | **Eff (%)** | **pEC50** | | **Eff (%)** | **pEC50** | **Eff(%)** | **name** |
| **15-HT1A** | **-** | **14** | **-** | | **24** | **6.6** | **100** | **8-OH-DPAT** |
| **5-HT1B** | **5.6** | **75** | **-** | | **20** | **8.2** | **100** | **5-CT** |
| **5-HT1D** | **5.3** | **132** | **-** | | **28** | **8.4** | **100** | **5-CT** |
| **5-HT1E** | **-** | **5** | **-** | | **5** | **6.7** | **100** | **BRL 54443** |
| **5-HT1F** | **-** | **30** | **-** | | **22** | **6.8** | **100** | **BRL 54443** |
| **5-HT2A** | **6.2** | **113** | **5.8** | | **139** | **7.1** | **100** | **5-CT** |
| **5-HT2B** | **6.2** | **75** | **5.3** | | **111** | **7.3** | **100** | **5-CT** |
| **5-HT2C** | **5.1** | **68** | **4.5** | | **84** | **7.2** | **100** | **5-CT** |
| **5-HT6** | **<5** | **52** | | **nd** | | **6.6** | **100** | **5-CT** |
| **5-HT7** | **-** | **7** | | **nd** | | **9.0** | **100** | **5-CT** |
| **D1** | **-** | **11** | | **nd** | | **7.3** | **100** | **SKF 38393** |
| **D2** | **6.1** | **36** | **-** | | ***77** | **9.2** | **100** | **Pergolide** |
| **D3** | **5.4** | **54** | | **nd** | | **9.9** | **100** | **Pergolide** |
| **D4** | **<5** | **40** | | **nd** | | **7.1** | **100** | **Pergolide** |
| **D5** | **-** | **10** | | **nd** | | **6.9** | **100** | **SKF 38393** |
| **H1** | **-** | **2** | **-** | | **3** | **6.8** | **100** | **Histamine** |
| **M1** | **-** | **5** | **-** | | **11** | **5.9** | **100** | **Carbachol** |
| **M2** | **-** | **9** | **-** | | **22** | **6.8** | **100** | **Carbachol** |
| **M3** | **-** | **0** | **-** | | **6** | **6.0** | **100** | **Carbachol** |
| **M4** | **-** | **2** | **-** | | **4** | **5.9** | **100** | **Carbachol** |
| **M5** | **-** | **-5** | | **nd** | | **6.4** | **100** | **Carbachol** |
| **α1A** | **-** | **15** | **-** | | **22** | **6.9** | **100** | **Phenylephrine** |
| **α1B** | **-** | **-1** | **-** | | **10** | **6.4** | **100** | **Phenylephrine** |
| **α2A** | **-** | **2** | **-** | | **5** | **7.9** | **100** | **UK 14,306** |
| **α2B** | **-** | **13** | **-** | | **8** | **7.0** | **100** | **UK 14,306** |
| **α2C** | **-** | **6** | **-** | | **13** | **6.9** | **100** | **UK 14,306** |
| **Transporter** | **pKi** | **Inh%** | **pKi** | | **Inh%** | **pKi** | **Inh%** | **name** |
| **SERT** | **-** | **26** | **-** | | **38** | **8.5** | **100** | **Fluoxetine** |
| **DAT** | **-** | **6** | **-** | | **5** | **8.4** | **100** | **Indatraline** |
| **NET** | **-** | **11** | **-** | | **14** | **8.4** | **100** | **Desipramine** |

All data was obtained from Receptor Selection and Amplification Technology (R-SAT^{™}) assays except for the transporter assays in which displacement of ^{12s}I-RTI-55 (for dopamine transporter (DAT)) or ³H-Imipramine (norepinephrine transporter (NET) and serotonin transporter (SERT)) was measured. A dash (-) indicates not calculated. nd indicates not done. Potency is reported as the negative logarithm of EC₅₀ in molar. Efficacy is reported as percent response of the indicated reference ligands. For receptors where (+) or (-)-OSU6162 showed no agonist activity, they were tested for functional antagonism of R-SAT^{™} responses, and in all cases they were inactive as antagonists (data not shown). Responses to references were at least 2.5 to 10 fold for all receptors. *response at 50 µM, the maximum concentration tested.

No agonist or antagonist activity of either enantiomer was evident at any of the muscarinic receptor subtypes, nor the alpha adrenergic receptor subtypes, nor H1 histamine receptors. No significant agonist or antagonist activity of (-)-OSU6162 at human D1 or D5 dopamine receptors were observed. Partial agonist activity of (-)-OSU6162 was evident at human D3 receptors, though it was less potent than at D2 receptors. Slight agonist activity of (-)-OSU-6162 was evident at D4 receptors, but mainly at 10 µM concentrations. Neither enantiomer had significant activity at 5-HT1 A, 1 E and 1 F receptors, nor (-)-OSU6162 at 5-HT6 or 5-HT7 receptors, whereas (-)-OSU6162 had agonist activity at 5-HT1 B and 5-HT1 D receptors, and to a lesser degree 5-HT2C receptors, but was approximately 3 to 10 fold less potent at these receptor subtypes than at 5-HT2A receptors. (-)-OSU6162 had partial agonist activity at 5-HT2B receptors, with similar potency to 5-HT2A receptors, whereas (+)-OSU6162 had greater efficacy and approximately 10-fold less potency at 5-HT2B receptors than (-)-OSU6162. In PI hydrolysis assays on 5-HT2B receptors we observed that (-)-OSU6162 had similar potency as in RSAT (pEC50 = 6.2) but significantly lower efficacy (24%, data not shown).

Prevention of reuptake of serotonin or dopamine could have been other factors contributing to the observed behavioral effects of (+)-OSU6162 or (-)-OSU6162. Thus, the binding affinities of (+)- and (-)-OSU6162 at the human dopamine transporter (DAT), serotonin transporter (SERT), and norepinephrine transporter (NET) were evaluated. Neither enantiomer of OSU6162 had significant binding affinity for any of these transporters (Table 3).

### Discussion

In the experiments described above the (-)-OSU6162-induced activation of monoamine-depleted mice was characterized, using specific receptor antagonists. Surprisingly, neither the dopamine D2-receptor antagonists haloperidol and raclopride, nor the dopamine D1 receptor antagonists SCH39166 and SCH23390, were able to antagonize the activation, whereas the selective 5-HT2A receptor antagonist M100907 effectively antagonized the response.

In parallel to the studies on (-)-OSU6162, comparative experiments on (+)-OSU6162 were performed. Surprisingly, in view of the fact that it had previously been stated not to induce any change in behavioral activity (Sonesson 1995 (as above)), it was found that the (+)-form was even more effective than the (-)-form in activating monoamine-depleted mice. Again, this action was resistant to treatment with D2 and D1 receptor antagonists but was effectively blocked by M100907.

These results strongly suggest that (-)- and (+)-OSU6162 are able to stimulate 5-HT2A receptors. Indeed, the results presented herein, using a battery of functional in vitro assays, show that both OSU6162 enantiomers have partial agonist effects on 5-HT2A receptors, the (+)-form displaying a higher intrinsic activity than the (-)-form. These results that tally with the more pronounced, M100907-reversible, activation observed following treatment with the (+)-enantiomer in monoamine-depleted mice.

(-)-OSU6162 and (+)-OSU6162 were profiled at a wide array of other monoaminergic targets including many of the other serotonin receptors, dopamine receptors, adrenergic receptors, H1 histamine receptors, and the serotonin, dopamine and norepinephrine transporters. Both enantiomers of OSU6162 had little to no activity at most of these targets. (-)-OSU6162 had significant agonist activity at 5-HT1 B and 5-HT1 D receptors, and to lesser degree 5-HT2C receptors, but was approximately 3 to 10 fold less potent at these receptor subtypes than at 5-HT2A receptors. (-)-OSU6162 had partial agonist activity at 5-HT2B receptors, at similar potencies as its actions at 5-HT2A receptors, whereas (+)-OSU6162 had greater efficacy but approximately 10-fold less potency at 5-HT2B receptors than (-)-OSU6162

The in vitro assays also showed that the agonist activity of (-)-OSU6162 and (+)-OSU6162 at 5-HT2A receptors occurred at similar concentrations as their actions at D2 receptors (see below), and this distinguishes them from other D2 partial agonists like (-)-3-PPP and aripiprazole that activate 5-HT2A receptors only at much higher concentrations than D2 receptors

In excellent agreement with the in vitro findings are also the observations on head twitches, a behavior adventitiously observed in monoamine-depleted mice following (-)-OSU6162 treatment. The head twitch behavior was investigated in a more systematic manner in drug-naive mice and it was found that both OSU6162 enantiomers produced head twitches but to a lesser degree than the nearly full 5-HT2 receptor agonist DOI. Furthermore, both OSU6162 enantiomers counteracted the DOI-induced head twitches, the (-)-enantiomer being considerably more effective than the (+)-enantiomer. Again, these results fit nicely with the in vitro data which demonstrate a higher intrinsic activity of (+)- than (-)-OSU6162 on 5-HT2A receptors.

The conclusion, based on the collected in vivo and in vitro data, that the OSU6162 enantiomers are partial agonists on the 5-HT2A receptors, tallies with the clinical observation that (-)-OSU6162, in contrast to the nearly full agonist DOI, has shown no hallucinogenic activity.

The conclusion that (-)- and (+)-OSU6162 are partial agonists on 5-HT2A receptors is compatible with the results in habituated rats: In habituated, low-active rats both OSU6162 enantiomers caused a dose-dependent stimulation of motor activity and this response was counteracted by treatment with M100907 - more effectively so with respect to the (+)-form - indicating a contribution of 5-HT2A receptor stimulation underlying the behavioral activation. A dopaminergic mechanism was also involved in the locomotor stimulation induced by (-) and (+)-OSU6162 in habituated rats, since haloperidol effectively antagonized the response.

Another finding from the *in vitro* functional assays, was that (-)-OSU6162 and (+)-OSU6162 behaved as D2 partial agonists. Interestingly, in these in vitro assays (-)-OSU6162 was found to be more potent than (+)-OSU6162, which is consistent with the former's higher potency to inhibit motor activity in drug naive mice and active (non-habituated) rats.

In conclusion, the present results indicate that both (-)-OSU6162 and (+)-OSU6162 act as stabilizers not only on dopaminergic, but also on serotonergic brain signaling. These discoveries have important implications for the potential clinical utility of both compounds, as well as for several of their congeners.

## Claims

1. 3-[3-(methylsulfonyl)phenyl]-1-propylpiperidine or a pharmaceutically acceptable salt thereof for use in the treatment and/or prevention of dementia selected from the group of disorders consisting of Alzheimer's disease, vascular dementia, frontotemporal dementia, semantic dementia and dementia with Lewy bodies, wherein 3-[3-(methylsulfonyl)phenyl]-1-propylpiperidine or the pharmaceutically acceptable salt thereof acts as a partial agonist on the 5-HT receptor.

2. 3-[3-(methylsulfonyl)phenyl]-1-propylpiperidine or a pharmaceutically acceptable salt thereof for use according to claim 1, wherein the 5-hydroxytryptamine receptor (5-HT receptor) is selected from the group consisting of 5HT₁, 5HT₂, 5HT₃, 5HT₄, 5HT₅, 5HT₆, and 5HT₇ receptors.

3. 3-[3-(methylsulfonyl)phenyl]-1-propylpiperidine or a pharmaceutically acceptable salt thereof for use according to claim 1 or 2, wherein the 5-hydroxytryptamine receptor (5-HT receptor) is selected from the group consisting of 5HT_{1A}, 5HT_{1B}, 5HT_{1D}, 5HT_{1E}, 5HT_{1F}, 5HT_{2A}, 5HT_{2B}, 5HT_{2C}, 5HT₃, 5HT₄, 5HT_{5A}, 5HT₆, and 5HT₇ receptors.

4. 3-[3-(methylsulfonyl)phenyl]-1-propylpiperidine or a pharmaceutically acceptable salt thereof for use according any one of the claims 1-3, wherein the 5-hydroxytryptamine receptor (5-HT receptor) is a 5HT₂ receptor.

5. 3-[3-(methylsulfonyl)phenyl]-1-propylpiperidine or a pharmaceutically acceptable salt thereof for use according to any one of the claims 1-4, wherein the 5-hydroxytryptamine receptor (5-HT receptor) is selected from the group consisting of 5HT_{2A}, 5HT_{2B}, and 5HT_{2C}.

6. 3-[3-(methylsulfonyl)phenyl]-1-propylpiperidine or a pharmaceutically acceptable salt thereof for use according to any one of the claims 1-5, wherein the 5-hydroxytryptamine receptor (5-HT receptor) is 5HT_{2A} or 5HT_{2B}.

7. 3-[3-(methylsulfonyl)phenyl]-1-propylpiperidine or a pharmaceutically acceptable salt thereof for use according to any one of the claims 1-6, wherein the compound is (3S)-3-[3-(methylsulfonyl)phenyl]-1-propylpiperidine or a pharmaceutically acceptable salt thereof.

8. 3-[3-(methylsulfonyl)phenyl]-1-propylpiperidine or a pharmaceutically acceptable salt thereof for use according to any one of the claims 1-6, wherein the compound is (3R)-3-[3-(methylsulfonyl)phenyl]-1-propylpiperidine or a pharmaceutically acceptable salt thereof.

## Patentansprüche

1. 3-[3-(Methylsulfonyl)phenyl]-1-propylpiperidin oder ein pharmazeutisch unbedenkliches Salz davon zur Verwendung bei der Behandlung und/oder Vorbeugung von Demenz, ausgewählt aus der Gruppe von Erkrankungen bestehend aus Morbus Alzheimer, vaskulärer Demenz, frontotemporaler Demenz, semantischer Demenz und Lewy-Körperchen-Demenz, wobei 3-[3-(Methylsulfonyl)phenyl]-1-propylpiperidin oder das pharmazeutisch unbedenkliche Salz davon als Teilagonist am 5-HT-Rezeptor wirkt.

2. 3-[3-(Methylsulfonyl)phenyl]-1-propylpiperidin oder ein pharmazeutisch unbedenkliches Salz davon zur Verwendung nach Anspruch 1, wobei der 5-Hydroxytryptamin-Rezeptor (5-HT-Rezeptor) aus der aus 5HT₁-, 5HT₂-, 5HT₃-, 5HT₄-, 5HT₅-, 5HT₆- und 5HT₇-Rezeptoren bestehenden Gruppe ausgewählt ist.

3. 3-[3-(Methylsulfonyl)phenyl]-1-propylpiperidin oder ein pharmazeutisch unbedenkliches Salz davon zur Verwendung nach Anspruch 1 oder 2, wobei der 5-Hydroxytryptamin-Rezeptor (5-HT-Rezeptor) aus der aus 5HT_{1A}-, 5HT_{1B}-, 5HT_{1D}-, 5HT_{1E}-, 5HT_{1F}-, 5HT_{2A}-, 5HT_{2B}-, 5HT_{2C}-, 5HT₃-, 5HT₄-, 5HT_{5A}-, 5HT₆-und 5HT₇-Rezeptoren bestehenden Gruppe ausgewählt ist.

4. 3-[3-(Methylsulfonyl)phenyl]-1-propylpiperidin oder ein pharmazeutisch unbedenkliches Salz davon zur Verwendung nach einem der Ansprüche 1-3, wobei es sich bei dem 5-Hydroxytryptamin-Rezeptor (5-HT-Rezeptor) um einen 5HT₂-Rezeptor handelt.

5. 3-[3-(Methylsulfonyl)phenyl]-1-propylpiperidin oder ein pharmazeutisch unbedenkliches Salz davon zur Verwendung nach einem der Ansprüche 1-4, wobei der 5-Hydroxytryptamin-Rezeptor (5-HT-Rezeptor) aus der aus 5HT_{2A}, 5HT_{2B} und 5HT_{2C} bestehenden Gruppe ausgewählt ist.

6. 3-[3-(Methylsulfonyl)phenyl]-1-propylpiperidin oder ein pharmazeutisch unbedenkliches Salz davon zur Verwendung nach einem der Ansprüche 1-5, wobei es sich bei dem 5-Hydroxytryptamin-Rezeptor (5-HT-Rezeptor) um 5HT_{2A} oder 5HT_{2B} handelt.

7. 3-[3-(Methylsulfonyl)phenyl]-1-propylpiperidin oder ein pharmazeutisch unbedenkliches Salz davon zur Verwendung nach einem der Ansprüche 1-6, wobei es sich bei der Verbindung um (3S)-3-[3-(Methylsulfonyl)phenyl]-1-propylpiperidin oder ein pharmazeutisch unbedenkliches Salz davon handelt.

8. 3-[3-(Methylsulfonyl)phenyl]-1-propylpiperidin oder ein pharmazeutisch unbedenkliches Salz davon zur Verwendung nach einem der Ansprüche 1-6, wobei es sich bei der Verbindung um (3R)-3-[3-(Methylsulfonyl)phenyl]-1-propylpiperidin oder ein pharmazeutisch unbedenkliches Salz davon handelt.

## Revendications

1. 3-[3-(Méthylsulfonyl)phényl]-1-propylpipéridine ou sel pharmaceutiquement acceptable de celle-ci pour utilisation dans le traitement et/ou la prévention de la démence choisie dans le groupe de troubles constitué de la maladie d'Alzheimer, la démence vasculaire, la démence fronto-temporale, la démence sémantique et la démence avec corps de Lewy, où la 3-[3-(méthylsulfonyl)phényl]-1-propylpipéridine ou le sel pharmaceutiquement acceptable de celle-ci agit comme un agoniste partiel sur le récepteur de 5-HT.

2. 3-[3-(Méthylsulfonyl)phényl]-1-propylpipéridine ou sel pharmaceutiquement acceptable de celle-ci pour utilisation selon la revendication 1, où le récepteur de 5-hydroxytryptamine (récepteur 5-HT) est choisi dans le groupe constitué des récepteurs 5HT₁, 5HT₂, 5HT₃, 5HT₄, 5HT₅, 5HT₆ et 5HT₇.

3. 3-[3-(Méthylsulfonyl)phényl]-1-propylpipéridine ou sel pharmaceutiquement acceptable de celle-ci pour utilisation selon la revendication 1 ou 2, où le récepteur de 5-hydroxytryptamine (récepteur 5-HT) est choisi dans le groupe constitué des récepteurs 5HT_{1A}, 5HT_{1B}, 5HT_{1D}, 5HT_{1E}, 5HT_{1F}, 5HT_{2A}, 5HT_{2B}, 5HT_{2C}, 5HT₃, 5HT₄, 5HT_{5A}, 5HT₆ et 5HT₇.

4. 3-[3-(Méthylsulfonyl)phényl]-1-propylpipéridine ou sel pharmaceutiquement acceptable de celle-ci pour utilisation selon l'une quelconque des revendications 1 à 3, où le récepteur de 5-hydroxytryptamine (récepteur 5-HT) est un récepteur 5HT₂.

5. 3-[3-(Méthylsulfonyl)phényl]-1-propylpipéridine ou sel pharmaceutiquement acceptable de celle-ci pour utilisation selon l'une quelconque des revendications 1 à 4, où le récepteur de 5-hydroxytryptamine (récepteur 5-HT) est choisi dans le groupe constitué de 5HT_{2A}, 5HT_{2B} et 5HT_{2C}.

6. 3-[3-(Méthylsulfonyl)phényl]-1-propylpipéridine ou sel pharmaceutiquement acceptable de celle-ci pour utilisation selon l'une quelconque des revendications 1 à 5, où le récepteur de 5-hydroxytryptamine (récepteur 5-HT) est 5HT_{2A} ou 5HT_{2B}.

7. 3-[3-(Méthylsulfonyl)phényl]-1-propylpipéridine ou sel pharmaceutiquement acceptable de celle-ci pour utilisation selon l'une quelconque des revendications 1 à 6, où le composé est la (3S)-3-[3-(méthylsulfonyl)phényl]-1-propylpipéridine ou un sel pharmaceutiquement acceptable de celle-ci.

8. 3-[3-(Méthylsulfonyl)phényl]-1-propylpipéridine ou sel pharmaceutiquement acceptable de celle-ci pour utilisation selon l'une quelconque des revendications 1 à 6, où le composé est la (3R)-3-[3-(méthylsulfonyl)phényl]-1-propylpipéridine ou un sel pharmaceutiquement acceptable de celle-ci.
